(19) Europäisches Patentamt
European Patent Office
Office européen des brevets

(11) **EP 4 066 816 A1**

(12) **EUROPEAN PATENT APPLICATION**
published in accordance with Art. 153(4) EPC

(43) Date of publication:
**05.10.2022 Bulletin 2022/40**

(21) Application number: **20893422.4**

(22) Date of filing: **27.11.2020**

(51) International Patent Classification (IPC):
*A61K 8/98* (2006.01)      *A61Q 19/00* (2006.01)
*A61Q 19/08* (2006.01)      *A61Q 19/02* (2006.01)
*A61Q 5/02* (2006.01)       *A61Q 7/00* (2006.01)
*A23L 33/10* (2016.01)      *A61K 35/20* (2006.01)
*A61P 17/02* (2006.01)

(52) Cooperative Patent Classification (CPC):
**A23L 33/10; A61K 8/98; A61K 35/20; A61P 17/02;
A61Q 5/02; A61Q 7/00; A61Q 19/00; A61Q 19/02;
A61Q 19/08**

(86) International application number:
**PCT/KR2020/017151**

(87) International publication number:
**WO 2021/107706 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.11.2019  KR 20190156058**

(71) Applicants:
• **Korea Institute of Science and Technology**
  **Seoul 02792 (KR)**
• **HB Advisors**
  **Yongsan-gu**
  **Seoul 04420 (KR)**

(72) Inventors:
• **KIM, Sun Hwa**
  **Seoul 02792 (KR)**
• **YANG, Yoosoo**
  **Seoul 02792 (KR)**
• **KIM, Hyo Suk**
  **Seoul 02792 (KR)**
• **KWAK, Gi-Jung**
  **Seoul 02792 (KR)**
• **JANG, Ye Ji**
  **Seoul 04420 (KR)**

(74) Representative: **Dehns**
**St. Bride's House**
**10 Salisbury Square**
**London EC4Y 8JD (GB)**

(54) **NOVEL USE OF MILK EXOSOMES**

(57)    The present invention relates to a novel use of milk exosomes, and more specifically, provides various uses of milk exosomes, such as in functional cosmetics, functional health food with various functions such as improving gut health and immunity, and drugs including a wound healing medication.

FIG. 7A

EP 4 066 816 A1

**Description**

**TECHNICAL FIELD**

The present invention relates to a novel use of milk exosomes and, more specifically, relates to various uses of milk exosomes in functional cosmetics, health functional foods, medicines, and the like.

**BACKGROUND ART**

Exosomes are vesicles with a size of several tens to hundreds of nanometers, made of double layers of phospholipids identical to the structure of the plasma membrane. Exosomes contain exosome cargos of proteins, nucleic acids (mRNA, miRNA, etc.) and the like, and in such exosome cargos, a wide range of signaling factors are included, and these signaling factors are known to be specific to cell types and differently regulated according to the environment of secretory cells. It is known that exosomes are intercellular signaling mediators secreted by cells, and various cellular signals transmitted through exosomes regulate cell behaviors, including activation, growth, migration, differentiation, dedifferentiation, apoptosis, and necrosis of targeted cells. It is known that exosomes contain specific genetic materials and bioactive factors according to the nature and state of their originating cells.

Since exosomes are basically originated from cells, they are biocompatible, unlike other nanoparticles, and due to their ability to carry or label a drug or a biologically active component inside or on the surface, there has been a continuous effort to utilize exosomes as a drug carrier or a raw material for cosmetics or drugs.

In this regard, Korean patent KR 2039302 discloses a cosmetic composition for strengthening and functionally improving skin barrier of the stratum cornea, comprising exosomes derived from stem cells as an active ingredient; Korean patent KR 1662405 discloses a pharmaceutical composition for treating cerebrovascular diseases, comprising exosomes derived from stem cells as an active ingredient; Korean patent KR 1894229 discloses a composition, comprising exosomes derived from deer antler stem cell culture as an active ingredient, for preventing or treating hair loss, or growing hair or promoting hair growth; and Patent WO 2016039356A1 discloses an anti-inflammatory agent comprising milk-derived exosomes as an active ingredient.

However, with regard to milk-derived exosomes, other than their use as an anti-inflammatory agent disclosed in WO2016039356A1, and use as a drug carrier disclosed by Munagala et al. (Cancer Lett., 371(1): 48-61, 2016), there is little known about other uses of milk-derived exosomes are.

Meanwhile, skin consists of the epidermis, which is a stratified squamous epithelium, the dermis composed of dense connective tissues, and the subcutaneous layer composed of loose connective tissues. The epidermis is located at the outermost layer of the skin, forming a water-proof protective membrane covering the surface of a body, and is composed of layers of squamous epithelial cells and the basal layer underneath. The epidermis contains no blood vessels, and only a slight amount of nerves in relation to the central nervous system. The main cell types constituting the epidermis include keratinocytes, melanocytes, Langerhans cells, and Merkel cells. The dermal layer is a skin layer underneath the epidermis that is composed of connective tissues, and provides a cushioning effect that protects the body from pressure and tension. The dermis is tightly connected to the epidermis via the basal layer, thereby supporting the epidermis and supplying nutrients, and contains a number of nerve endings sensing touch and heat.

The skin is gradually damaged by advancing age, or UV rays, external pollutants, and stress, and as the function to protect the skin from such factors decreases, cell protection and proliferation capabilities also decrease. In this case, our body initiates the skin regeneration process for damaged skin. Skin regeneration is any reaction of tissue against damage and is a complex biological process comprising chemotaxis, cell differentiation and replication, synthesis of matrix protein, angiogenesis and reconstitution of wound, as a series of tissue repair processes (Steed, D. L. et al., Clin. Plast. Surg. 25: 397, 1998).

When the skin is damaged, the process of cell proliferation follows inflammation reactions, which lasts from 2 days to 3 weeks after the skin damage. During this period, fibroblasts deposits collagen, filling the damaged skin, proliferation of fibroblasts is induced, and reconstitution of new cells and interstitial constituents takes place in the wound area. This proliferation process, also known as granulation process, lasts from 2 days to 3 weeks after the formation of wound. In the young skin by new cells, activities of skin cells are high such that fibroblasts in the dermis are highly active in collagen synthesis and metabolism, which is important for skin firmness and inhibition of wrinkle formation. In addition, the synthesis of GAGs (glucosamino-glycans), which is a glycoprotein containing hyaluronic acid that is essential for skin moisturization, is also active, thus imparting firmness, moisture, and elasticity to the skin. Also, in the epidermal layer, basal layer cells actively proliferate, and a balanced production of adhesion proteins such as laminin, integrin, and desmosome that enhance connections between skin cells, takes place, thus strengthening cell tissues and keeping the skin healthy.

However, since the skin regeneration capability decreases with advancing age, when skin damage occurs by UV rays, pollutants or stress, skin regeneration takes place incompletely. Thus, the skin loses firmness, forms wrinkles, undergoes discolorations and hyperpigmentation such as age spots, blemishes, freckles and dark spots, and loses water retention capability.

Therefore, in order to keep firm, moist and healthy skin, restoring the skin regeneration capability that decreases with advancing age may be the most important. Prior art pertaining to a functional cosmetic composition for such skin regeneration purposes include: Korean patent KR 1888258 pertaining to a composition for skin regeneration, comprising an essential oil extracted from Daisy fleabane flowers as an active ingredient; Korean patent KR 1422690 pertaining to a composition for skin regeneration comprising culture secretions of angiogenic progenitor cells derived from embryonic stem cells; and Korean patent KR 1663912 pertaining to a cosmetic composition for skin whitening, skin wrinkle reduction, or skin regeneration, comprising exosomes derived from human adipose-derived stem cells as an active ingredient.

However, the above prior art have issues such as an excessive production cost and unchecked in vivo stability and safety.

Meanwhile, immunity is a self-defense response that physiologically distinguishes exogenous and endogenous foreign materials in the body, and eliminates and metabolizes them. Immunity can be classified as innate immunity, which is initial immunity, and acquired immunity, which is adaptive immunity. In the initial immunity, activities of macrophages and natural killer cells (NK cells) protect the host by suppressing foreign materials (pathogens), and here, the macrophages produce and release activity marker TNF-á, while engulfing foreign materials, and the NK cells produce and release activity marker, perforin, to kill pathogen-infected cells. Subsequently, cytotoxic T lymphocytes, helper T lymphocytes, and B lymphocytes involved in acquired immunity are activated to kill infected cells or produce antibodies to protect the host. Cytotoxic T lymphocytes, just as NK cells, produce and release a large amount of perforin to kill pathogen-infected cells, and B lymphocytes produce antibodies, dependently or independently on helper T lymphocytes, to protect the host. Inflammatory cytokines such as IL-6, IL-8, and TNF-á, are substances that mediate immunity and are known to be involved in the initial immunity in particular. Furthermore, since the innate immunity plays an extremely important role as a defense mechanism against cancer cells, cellular therapy products utilizing the activation of innate immunity, such as NK cells and T cells as a mechanism of action have garnered more attention in recent years.

In general, in the case of immunodeficiency, resistance to infection is compromised, and patients with antibody deficiencies are unable to defend against bacterial infections, and the phagocytic capability of neutrophils is also compromised. Moreover, in such a case, activation of the complement system is also compromised, failing to produce leukocyte migration factors and the like, subsequently increasing the rate of inflammations and causing viremia, spreading to the central nervous system and other sites. Moreover, in case of a cancer patient, not only cancer cells but also normal cells are affected during the process of chemotherapy or radiation therapy, thus causing an adverse effect that drastically decreases the patient's immunity.

In this context, there is a need for development of an immunity enhancer capable of enhancing immunity, or functional health food for strengthening immunity, for the prevention or treatment of cancer or infectious diseases.

Meanwhile, according to health insurance medical expenses payment data surveyed by Health Insurance Policy Research Institute of National Health Insurance Corporation from 2001 to 2008, the number of patients actually treated for 'hair loss' was 103,000 in 2001, 142,000 in 2005, and 165,000 in 2008, showing a 60% increase over the past 7 years. By age, the number of patients in their 20's to 40's was 114,000, which accounts 69.5% of the total number of patients, and the number of patients in their 10s or younger was 22,000 or more. As of 2008, among the actually treated patients, 84,000 were men and 80,000 were women, showing that there were slightly more men than women, and as of 2008, the specific types of 'hair loss' among the actually treated patients in natural health insurance include alopecia areata (130,000), cicatricial alopecia (2,000), androgenic alopecia (9,000), and other nonscarring hair loss (8,000). Meanwhile, according to the data of the International Hair and Cosmetics Research Forum in June 2003, there are 250 millions of hair loss patients, and the incidence of hair loss between the age of 24 to 50 was 30 to 65%. As of 2008, there are about 300 million people with hair loss in China, and 30% of the male population in their 30s and 50% of the male population in their 50s show signs of hair loss, and the number of hair loss patients increases by 10-15% every year. According to data surveyed by businesses selling wigs and performing hair transplant surgery in 2007 in Japan, the incidence of hair loss in Japanese population was 26.5%, and the number of patients with hair loss was estimated to be about 12.93 million.

Currently, preparations for hair loss treatment are largely classified into drugs, quasi-drugs, and cosmetics. Prescription drugs that can only be purchased with a doctor's prescription include 'Propecia' developed and marketed by Merck in the US. Finasteride, the main ingredient of Propecia, was approved as a hair loss treatment medication by the US FDA in December 1997. Finasteride is a drug that inhibits 5-alphareductase, which converts testosterone to dihydrotestosterone (DHT), and plays a role in growing vellus hair into thick and long hair. Although effective in alleviating hair loss in the short term, Finasteride is accompanied by side effects such as erectile dysfunction, decreased sexual function, and enlarged breasts in men. Over-the-counter drugs that have been recognized for their safety and effectiveness and thus can be purchased without a doctor's prescription include Minoxidil. Minoxidil was approved by the FDA in December 1997 as the first topical hair loss treatment medication. Although this medication has the effect of promoting hair growth by improving blood circulation and opening potassium channels, it may cause local reactions such as itchiness and rash may occur, and may cause tachycardia and the like.

Among products that have been approved for hair loss prevention and hair growth function by the Korean Food and Drug Administration, quasi-drug products that can be sold at supermarkets or convenience stores according to the public notice of the Minister of Health and Welfare include 'Hair Power Competent' by CJ Lion and 'Hair Tonic' by Moracle, 'Mo & More' by LG Household & Health Care, and as cosmetics, shampoos or products used for scalp or hair to maintain or promote the health of skin and hair are sold.

The human hair loss cycle is largely divided into the growing phase (anagen), the transitional phase (catagen), and the resting phase (telogen). The anagen phase is a phase in which hair follicle dermal papilla cells are active, cells are dividing rapidly, and the hair grows at a fast rate. The duration of the anagen phase, although it varies on the type of hair, is about 3-6 years for the head hair. Anagen hair accounts for 80 to 90% of total hair, and people who are experiencing hair loss tend to have a hair cycle with a shortened anagen phase and a prolonged telogen phase, decreasing the portion of anagen hair in the total hair. The catagen phase is a phase in which the anagen phase of hair ends and hair generation gradually slows down, with cell division and growth eventually coming to an end. The catagen phase lasts about 1-1.5 months, and about 1% of the total hair belongs to this phase. The telogen phase is the last stage of growth, in which the hair follicles and dermal papilla are completely separated, and the hair follicles are atrophied, and the hair follicles move further upwards, causing the hair to fall out. The telogen phase lasts about 3-4 months, and 4-14% of the total hair belong to this phase. As the telogen phase ends and the dermal papilla is active again, dermal papilla of new hair are formed, and hair in the telogen phase is pushed out and comes out of the scalp completely.

Since conventional hair loss treatment agents or compositions for hair growth have their efficacy not fully verified, or are accompanied by various side effects, there is an urgent need for the development of a safer and more efficient hair loss treatment agent or hair growth agent.

## DISCLOSURE OF THE INVENTION

### TECHNICAL PROBLEM

The present invention has been conceived of to address various issues including the aforementioned issues, and the objective of the present invention is to provide a novel use of more efficient and inexpensive milk-derived exosomes in various cosmetics such as a functional cosmetic composition for skin regeneration, food, and drugs. However, the scope of the present invention is not limited to the above objective.

### TECHNICAL SOLUTION

According to one aspect of the present invention, provided is a cosmetic composition for skin regeneration, including exosomes isolated from milk or goat milk as an active ingredient.

According to another aspect of the present invention, provided is a cosmetic composition for wrinkle reduction, including exosomes isolated from milk or goat milk as an active ingredient.

According to another aspect of the present invention, provided is a cosmetic composition for skin whitening, including exosomes isolated from milk or goat milk as an active ingredient.

According to another aspect of the present invention, provided is a functional cosmetic product for hair for alleviating and preventing hair loss, including exosomes isolated from milk or goat milk as an active ingredient.

According to another aspect of the present invention, provided is a functional health food including exosomes isolated from milk or goat milk as an active ingredient.

According to another aspect of the present invention, provided is a pharmaceutical composition for wound treatment, including exosomes isolated from milk or goat milk as an active ingredient.

According to another aspect of the present invention, provided is a pharmaceutical composition for treating hair loss or growing hair or promoting hair growth, including exosomes isolated from milk or goat milk as an active ingredient.

According to another aspect of the present invention, provided is a method of accelerating regeneration of wounded skin of a subject, the method including applying a therapeutically effective amount of the exosomes isolated from milk or goat milk to a wounded skin of the subject.

According to one aspect of the present invention, provided is a method of enhancing immunity of a subject, the method including administering a therapeutically effective amount of the exosomes isolated from milk or goat milk to the subject.

According to one aspect of the present invention, provided is a method of preventing or treating hair loss of a subject, the method including administering a therapeutically effective amount of the exosomes isolated from milk or goat milk to the subject.

According to another aspect of the present invention, a method of reducing skin wrinkles of a subject, the method including administering a therapeutically effective amount of the exosomes isolated from milk or goat milk to the subject.

According to another aspect of the present invention, a method of whitening the skin of a subject, the method including administering a therapeutically effective amount of the exosomes isolated from milk or goat milk to the subject.

According to another aspect of the present invention, provided is a use of exosomes isolated from milk or goat milk in the preparation of a wound healing agent.

According to another aspect of the present invention, provided is a use of exosomes isolated from milk or goat milk in the preparation of a cosmetics product for reducing skin wrinkles.

According to another aspect of the present invention, provided is a use of exosomes isolated from milk or goat milk in the preparation of a cosmetics product for whitening.

According to another aspect of the present invention, provided is a use of exosomes isolated from milk or goat milk in the preparation of an agent for preventing and treating hair loss.

According to another aspect of the present invention, provided is a use of exosomes isolated from milk or goat milk in the preparation of a hair growth agent.

According to another aspect of the present invention, provided is a use of exosomes isolated from milk or goat milk for a composition for improving immunity.

According to another aspect of the present invention, provided is a method of isolating exosomes from milk or goat milk, the method including: a first centrifugation step of removing fat and cells from milk or goat milk by centrifugation; a filter filtration step of filtering the centrifuged milk or goat milk with a strainer having a mesh size of 20 to 60 $\mu$m, to further remove fat and cells therefrom; a dilution step of diluting the filtered milk or goat milk by adding an equal volume of distilled water thereto; an isoelectric precipitation step of adding an acid to the diluted milk or goat milk to adjust the pH to 4 to 5, thereby precipitating casein in the milk or goat milk; a secondary centrifugation step of additionally centrifuging the milk or goat milk in which casein has been precipitated, and collecting supernatant therefrom; and a filtration step of filtering the collected supernatant by a filter of 0.2 $\mu$m.

## ADVANTAGEOUS EFFECTS

A cosmetic composition for skin regeneration according to an example of the present invention not only has a skin regeneration effect significantly higher than that of regular exosomes isolated from cell culture media, but also has a low production cost, a significantly high yield, and a high material stability, and thus, can be extremely economically produced. However, the scope of the present invention is not limited to the above-mentioned effects.

## BRIEF DESCRIPTION OF THE DRAWINGS

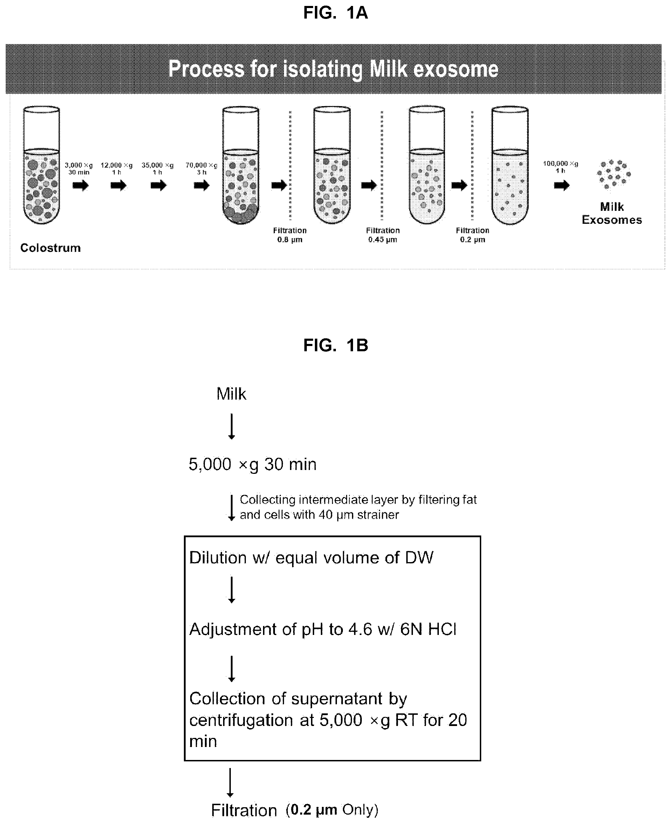

FIG. 1 is a flowchart schematically showing a process of isolating exosomes from milk or colostrum according to an example of the present invention.

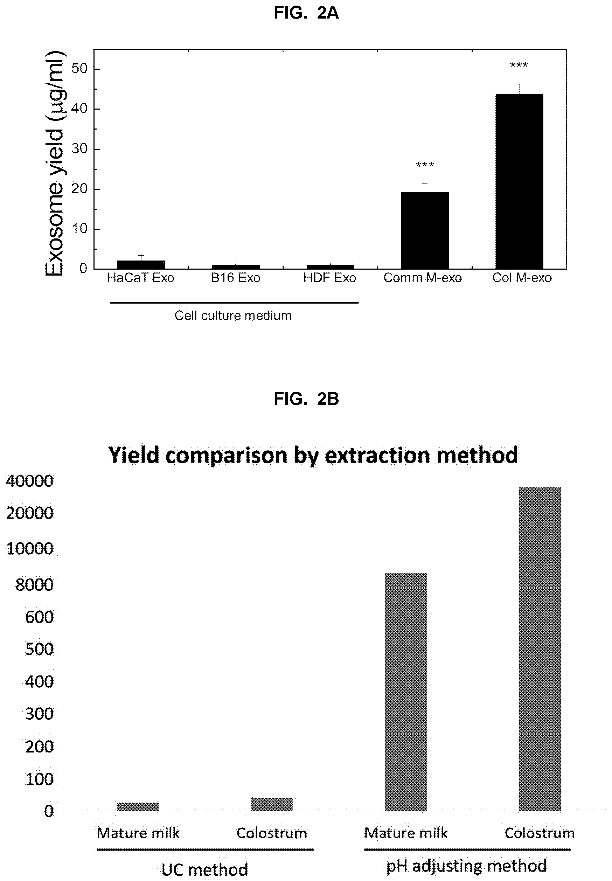

FIG. 2 is a graph showing yields of commercial milk-and colostrum-derived exosomes isolated according to an example of the present invention, and exosomes isolated from HEK293 cells as a control group.

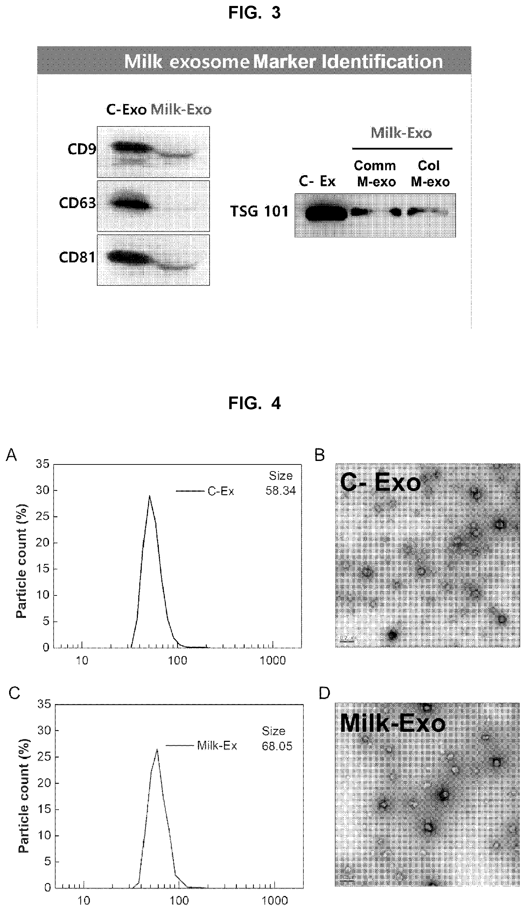

FIG. 3 is a series of photographs showing a result of Western blot analysis of exosome surface markers of commercial milk- and colostrum-derived exosomes isolated according to an example of the present invention, and exosomes isolated from HEK293 cells as a control group.

FIG. 4 shows histograms (A and C) showing results of dynamic light scattering analysis of particle sizes of milk-derived exosomes (A and B) isolated according to an example of the present invention, exosomes (C and D) isolated from HEK293 cells as a control group, and photographs (B and D) of the exosomes captured by a transmission electron microscope.

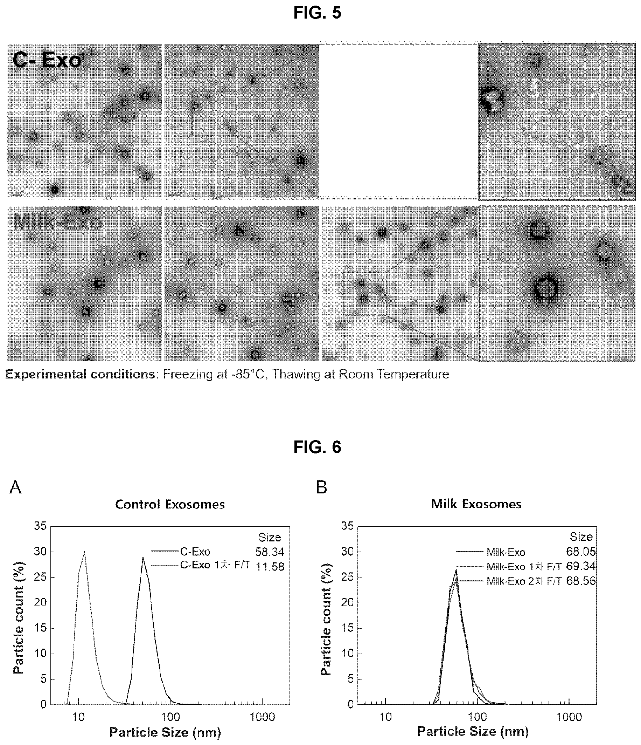

FIG. 5 is a series of photographs captured by a transmission electron microscope, of shapes of milk-derived exosomes isolated according to an example of the present invention (top) and HEK293-derived exosomes as a control group (bottom) before and after two freeze/thaw cycles. The red boxes in the photographs represent expanded areas on the right.

FIG. 6 is a series of histograms showing a result of dynamic light scattering analysis of particle size distributions of milk-derived exosomes isolated according to an example of the present invention (A) and HEK293-derived exosomes as a control group (B) before and after two freeze/thaw cycles.

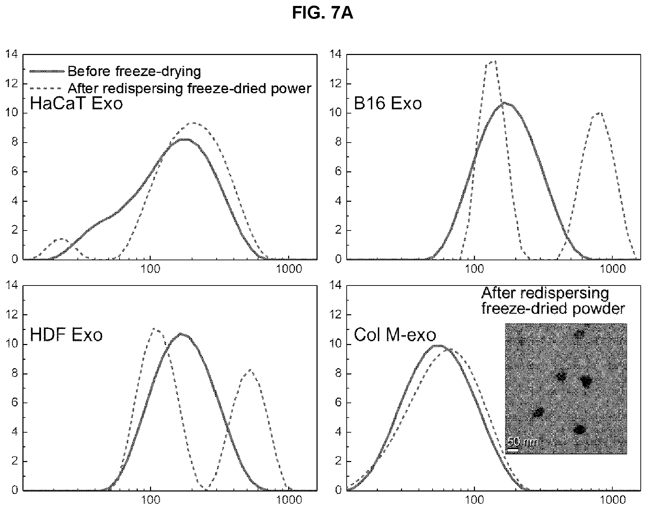

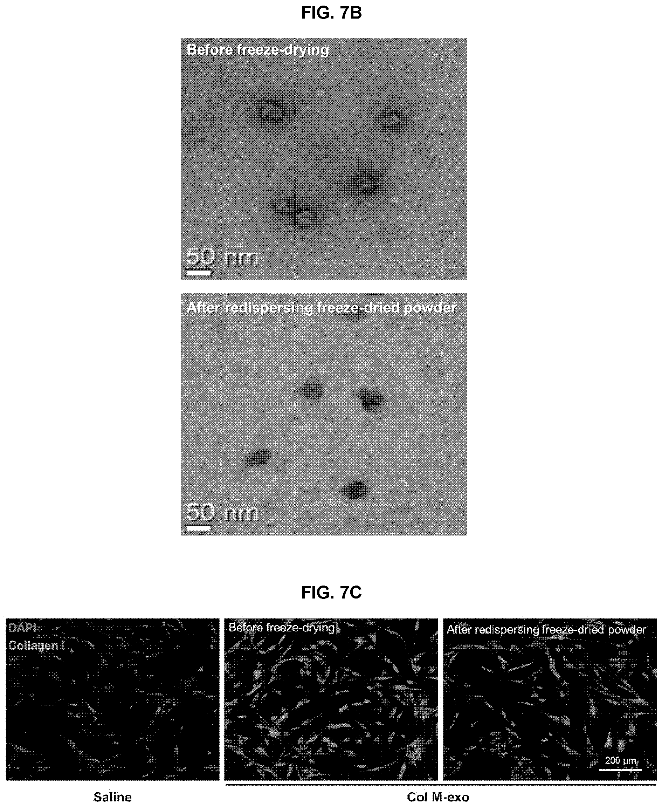

FIG. 7 represents a graph (A) representing an average particle size of milk-derived exosomes isolated according to an example of the present invention, measured on each day during storage at 4°C for 7 days, and a series of histograms (B) showing a result of dynamic light scattering analysis of particle size distributions of exosomes isolated from HEK293 cells, which is a control group (top), and milk-derived exosomes isolated according to an example of the present invention (top) before storage (Day 0) and after 7 days (Day 7) when stored at 4°C for 7 days.

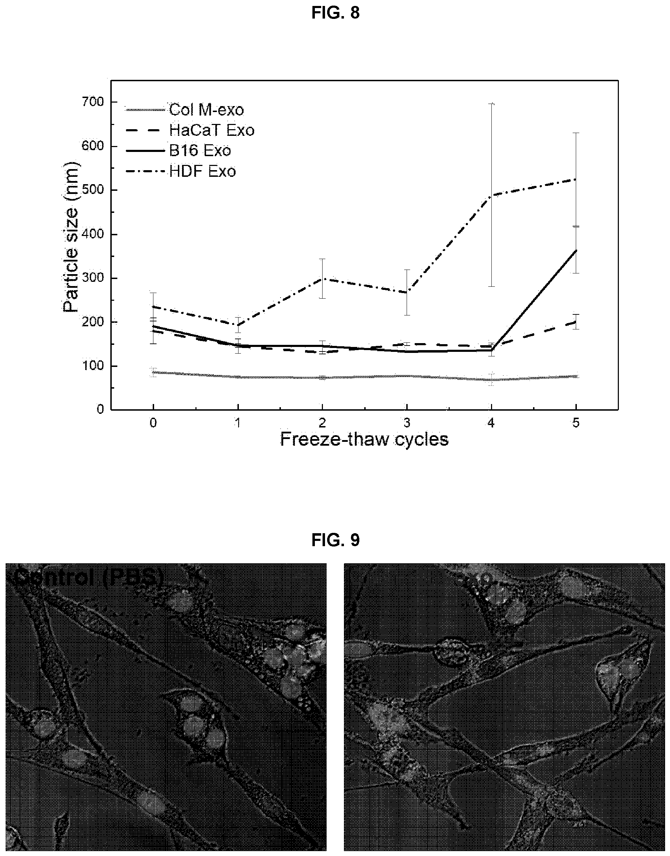

FIG. 8 is a fluorescent microscopy image (right) showing that milk-derived exosomes isolated according to an example of the present invention, when administered, are absorbed into human dermal fibroblasts. The left image is of a PBS-treated group, which is a control group. Blue color represents a fluorescent signal of DAPI for nuclear counterstaining.

FIG. 9 is a series of graphs showing a degree of cell proliferation measured after administering commercial milk- and colostrum-derived exosomes isolated according to an example of the present invention, respectively to human keratinocytes (HaCaT, A) and human dermal fibroblasts (HDF, B). The control group shows the degree of proliferation of cells only treated with PBS buffer. The proliferation rate represents a relative percentage when the proliferation rate of the control group is defined as 100%.

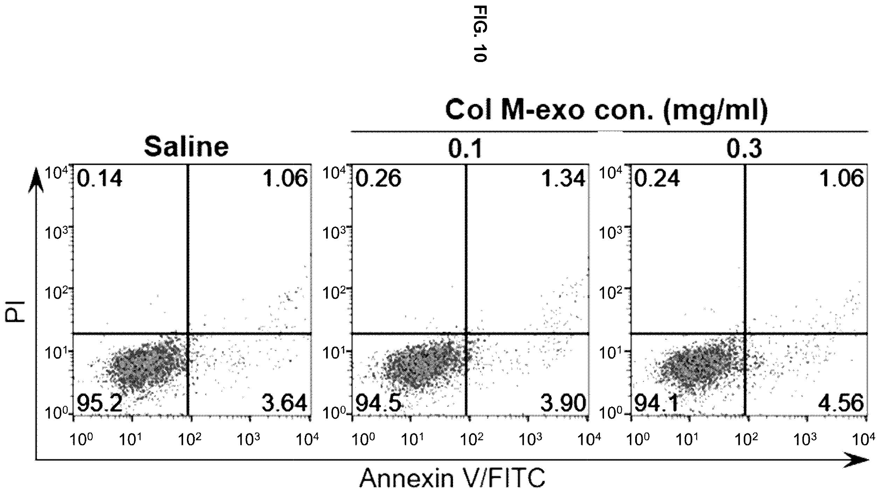

FIG. 10 is a series of images (A and C) showing the degree of cell migration observed after administering commercial milk- and colostrum-derived exosomes isolated according to an example of the present invention to scratchinduced human keratinocytes (HaCaT, A) and human dermal fibroblasts (HDF, B), and a series of graphs (B and D) of a degree of cell migration quantified from the results of A and C.

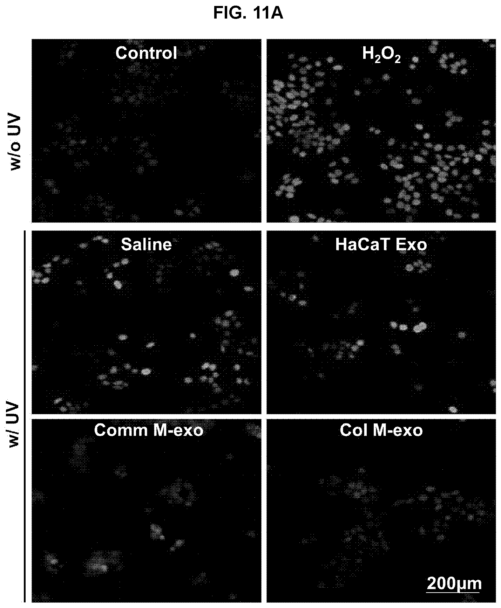

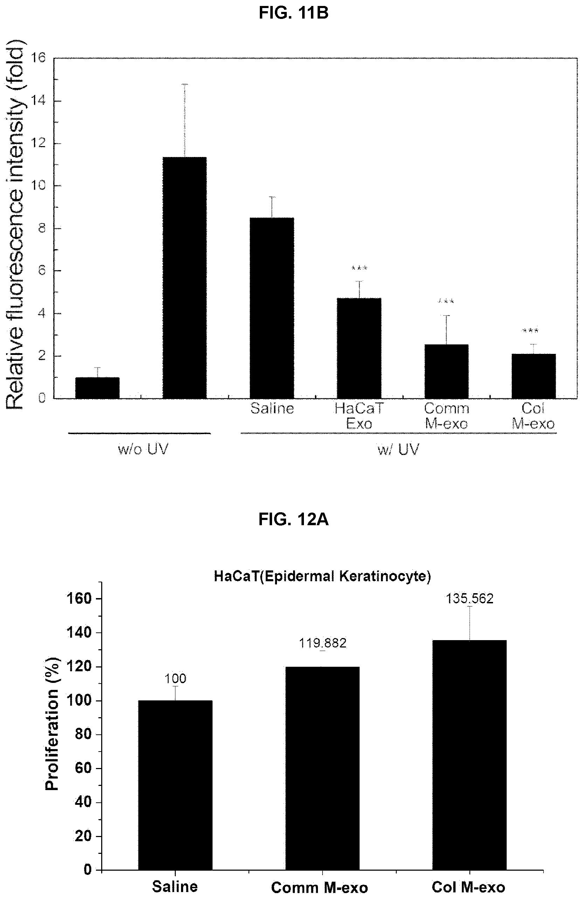

FIG. 11a shows a series of fluorescence microscopy images showing the result of analysis, made by capturing an image of fluorescence intensity by DCF-DA, which expresses fluorescence by reacting with reactive oxygen species, on the level of reactive oxygen species formation in human dermal fibroblasts damaged by UV radiation, after treatment with commercial milk-derived exosomes (Comm M-exo) and colostrum exosomes (Col M-exo) according to an example of the present invention. FIG. 11b is a graph showing quantified relative fluorescence intensity identified in FIG. 11a.

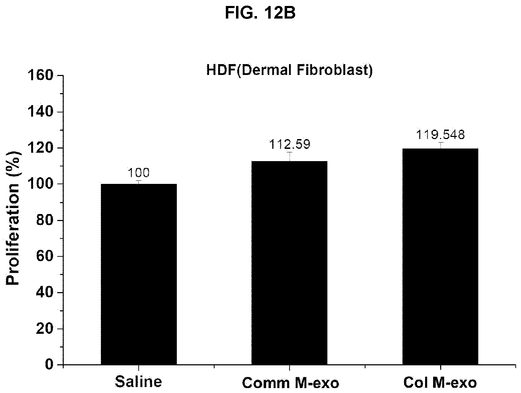

FIG. 12a shows a graph of cell proliferation rates in human keratinocytes (HaCaT), measured after treated respectively with exosomes derived from commercial milk and colostrum isolated according to an example of the present invention. FIG. 12b shows a series of graphs showing cell proliferation rates in human dermal fibroblasts (HDF), measured after treated respectively with exosomes derived from commercial milk and colostrum isolated according to an example of the present invention. The control group represents the degree of proliferation of cells treated with only PBS buffer, and the proliferation rate represents a relative percentage when the growth rate of the control group is set to 100%.

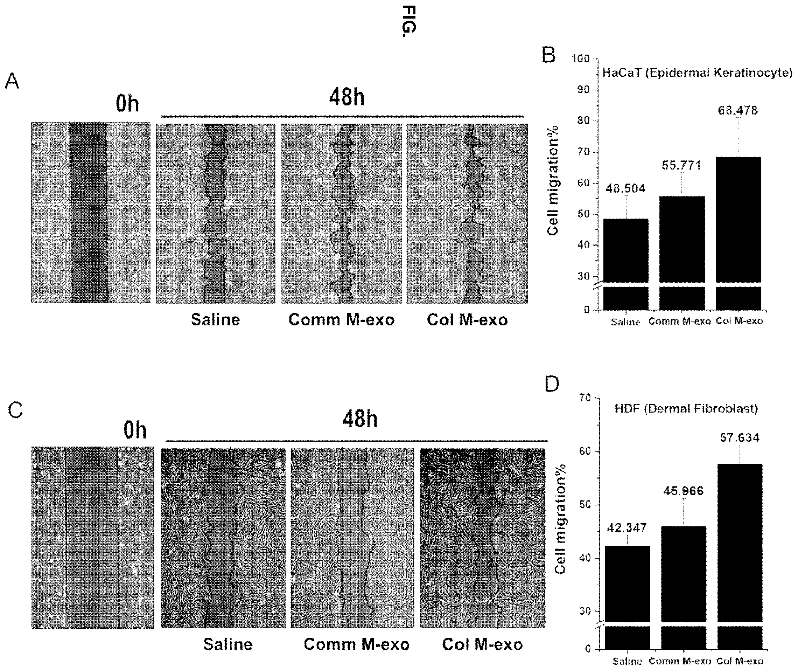

FIG. 13 shows a series of images (A and C) showing the degree of cell migration observed after administering commercial milk- and colostrum-derived exosomes isolated according to an example of the present invention to scratchinduced human keratinocytes (HaCaT, A) and human dermal fibroblasts (HDF, B), and a series of graphs (B and D) showing the degree of cell migration quantified from the results of A and C.

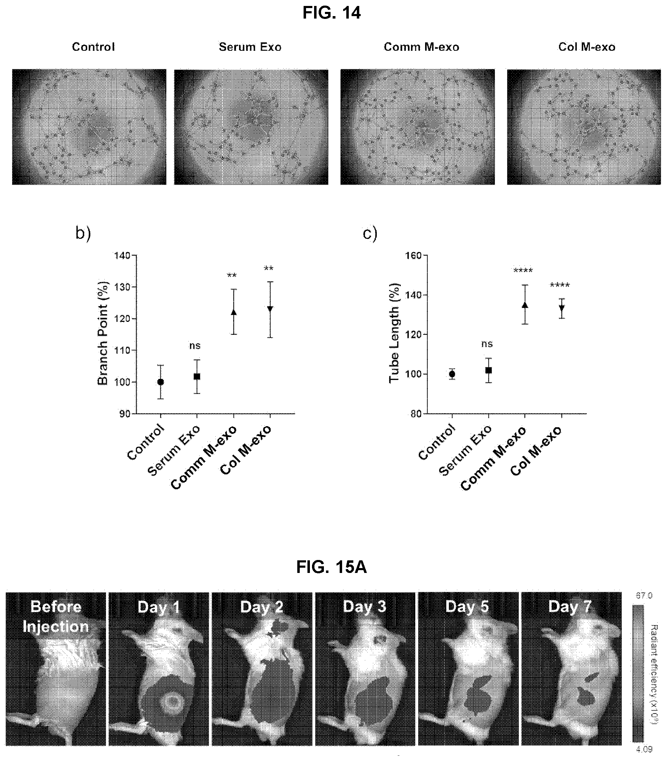

FIG. 14 shows (a) a series of images captured by a microscope, showing the degree of tube formation in murine endothelial cells (SVEC4-10) in three-dimensional culture in Matrigel, after treated with commercial milk-derived exosomes (Comm M-exo) and colostrum exosomes (Col M-exo) according to an example of the present invention, and a series of graphs showing (b) the number of branch points observed in (a), and (c) the length of tubes formed.

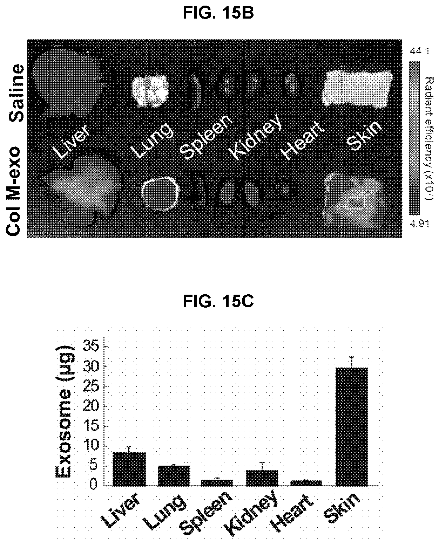

FIG. 15a shows a series of images captured by whole-body in vivo fluorescence imaging, showing in vivo distribution of colostrum exosomes according to an example of the present invention, labeled with a fluorescent material (Flamma 675) and subcutaneously injected. FIG. 15b is a photograph captured by an in vivo imaging system, showing fluorescence distribution in organs after sacrificing the experiment animal on the second day of exosome injection and harvesting the main organs. FIG. 15c is a graph of the quantified result of FIG. 15b.

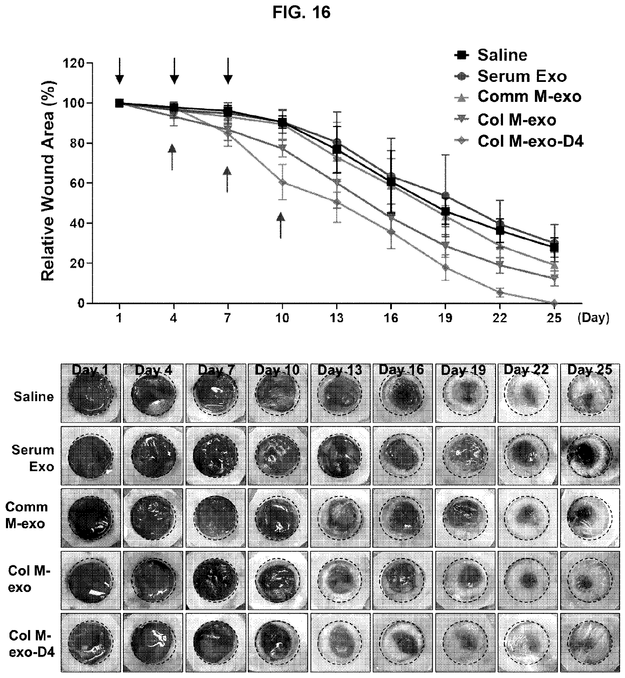

FIG. 16 shows a graph (top) showing the degree of wound healing recorded over time after applying commercial milk-derived exosomes (Comm M-exo) and colostrum exosomes (Col M-exo) according to an example of the present invention to a wound site of a wound model mouse, and a series of photographs directly captured of the wound site. For the control group, the group administered only with physiological saline (Saline) and exosomes obtained from serum (Serum Exo) were used, and the colostrum exosome group was divided into the group administered on Days 1, 4, and 7 (Col M-exo) and the group administered at Days 4, 7, and 10 (Col M-exo-D4).

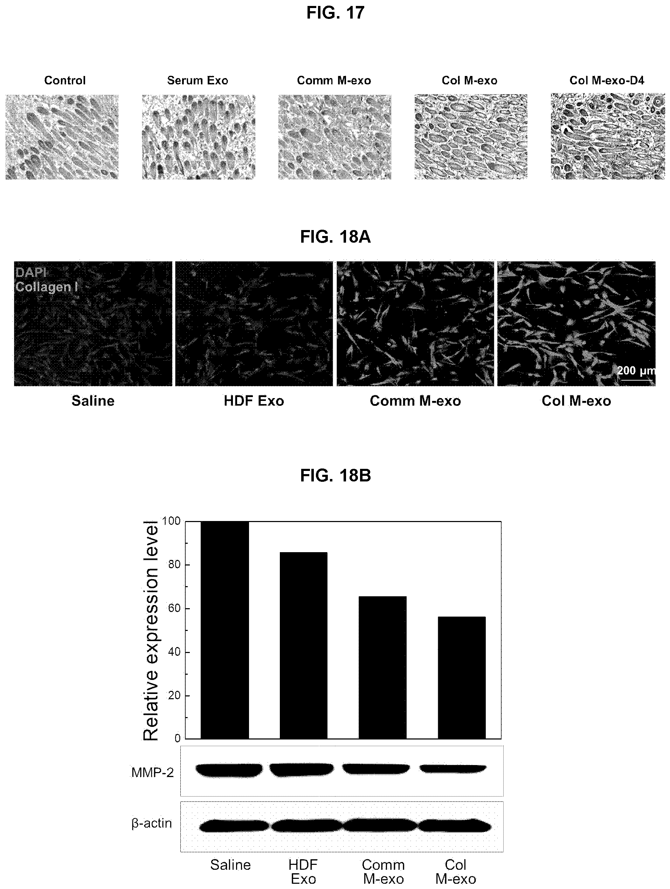

FIG. 17 is a series of photographs showing the result of an immunohistochemical assay using an anti-elastin antibody performed on a tissue section excised from the wound site after sacrificing the experiment animal of FIG. 16.

FIG. 18a is a series of fluorescence microscopic photographs showing the expression level of type 1 collagen protein, analyzed by immunofluorescence assay, in human dermal fibroblasts (HDF) after treatment with commercial milk-derived exosomes (Com M-exo) and colostrum exosomes (Col M-exo) according to an example of the present invention. FIG. 18b is a graph showing the expression level of matrix metalloproteinase-2 (MMP-2), analyzed by Western blot, in human dermal fibroblasts (HDF) after treatment with commercial milk-derived exosomes (Com M-exo) and colostrum exosomes (Col M-exo) according to an example of the present invention. The control groups are a saline-treated group (Saline) and a HDF-derived exosomes-treated group.

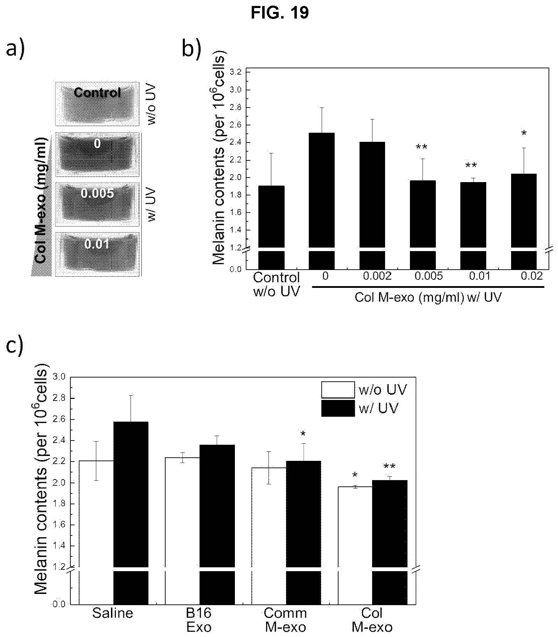

FIG. 19 shows (a) a series of photographs of melanin pigment extracted, 24 hours after the treatment, from melanoma cells treated or untreated with UV radiation, treated with various concentrations of colostrum exosomes (Col M-exo) according to an example of the present invention, (b) a graph showing the result of measuring the amount of melanin pigment per 10×6 cells, and (c) a graph showing the result of comparing the melanin content between the groups treated with commercial milk-derived exosomes (Comm M-exo) and colostrum exosomes (Col M-exo) according to an example of the present invention, and the control groups (Saline, B16-derived exosome treatment (B16 Exo)) .

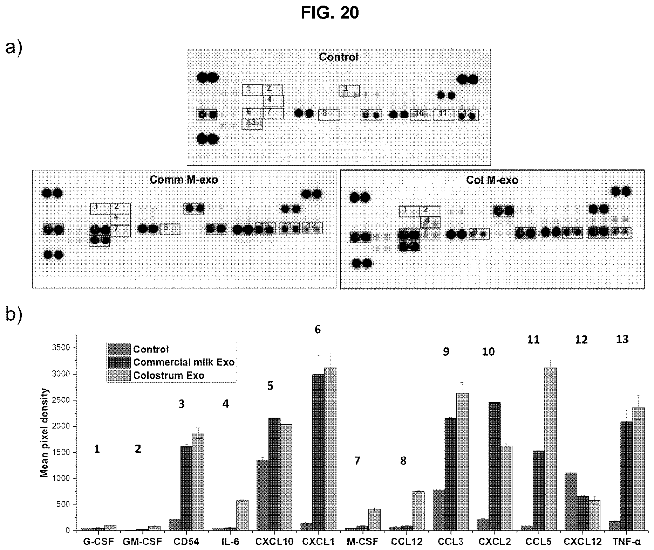

FIG. 20 shows (a) a photograph showing the expression level of various cytokines, analyzed by a dot blot assay 24 hours after the treatment, in bone marrow-derived macrophages (BMDM) treated with commercial milk-derived exosomes (Comm M-exo) and colostrum exosomes (Col M-exo) according to an example of the present invention, and (b) a graph showing the quantified result thereof.

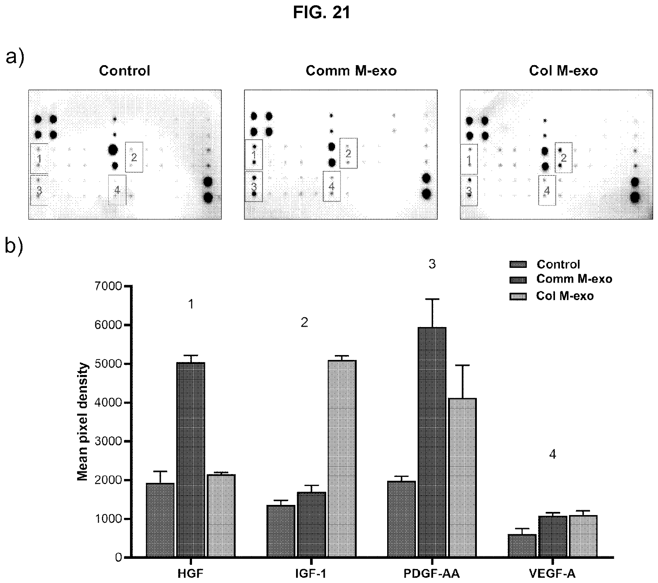

FIG. 21 shows (a) a result of a dot blot assay comparing the expression level of various growth factors involved in tissue regeneration and angiogenesis in NIH-3T3 fibroblasts treated with commercial milk-derived exosomes (Comm M-exo) and colostrum exosomes (Col M-exo), respectively, and (b) a graph showing the quantified result of the dot blot assay.

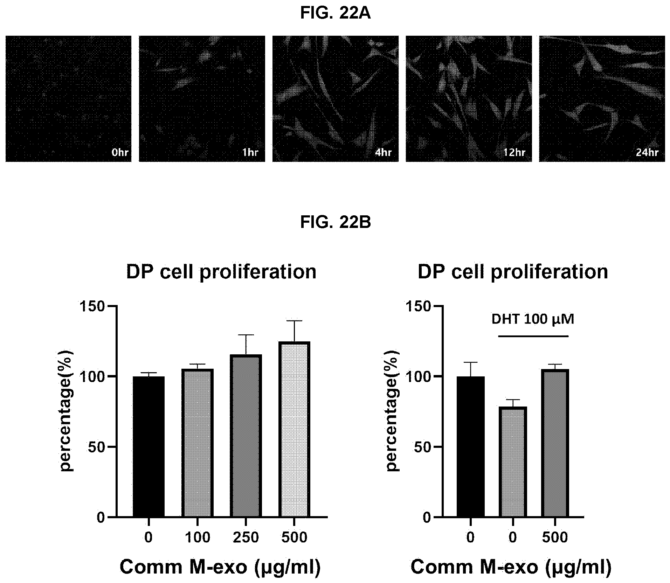

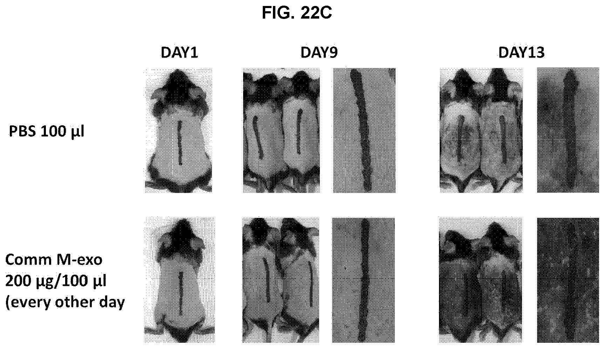

FIG. 22a shows a series of fluorescence microscopic images showing the result of observing the pattern of intracellular uptake of exosomes at different treatment durations in human dermal papilla cells treated with commercial milk-derived exosomes (Comm M-exo) according to an example of the present invention. FIG. 22B shows a graph (left) showing the result of analyzing the influence of colostrum exosomes on the proliferation of dermal papilla cells at different colostrum exosome treatment concentrations in human dermal papilla cells treated with colostrum exosomes according to an example of the present invention, and a graph (right) showing the result of analyzing difference in the degree of proliferation of human dermal papilla cells with and without treatment of a hair loss-inducing substance. FIG. 22C shows a series of photographs showing the result of observing the degree of hair growth over time after administering via transdermal administration, to shaved test animals, commercial milk-derived exosomes (Comm M-exo) according to an example of the present invention.

## MODE FOR CARRYING OUT THE INVENTION

### Definitions of the Terms

The terms used herein are defined as follows.

The term "exosome" used herein refers to a nanosized cell-derived vesicle that may be present in all types of biological fluids including blood, urine, and cell culture media. The size of exosomes is known to be between 30 nm to 100 nm and are secreted from cells from the fusion of multivesicular bodies with the plasma membrane, or directly secreted through the plasma membrane. Exosomes are known to play an important role in various processes such as coagulation, intercellular signal transduction, and management of metabolites. In this context, the term "milk exosomes" used herein refers to exosomes derived from milk of mammals, such as commercial milk and colostrum. Likewise, the term "colostrum exosomes" refers to milk exosomes derived from milk produced by the mother immediately after the birth of an offspring.

The term "milk" as used herein primarily refers to cow milk, but includes milk from other mammals, such as a horse, a sheep, a milk goat (goat), and a camel, as functional equivalents. Considering acquisition availability, cost, and the like, commercial cow milk is the most preferable, and commercial "goat milk" produced from milk goat (goat) can be an excellent alternative. In particular, goat milk has a similar composition as breast milk and is thus easy to digest, and has a high nutritional value, and as such, is recognized as premium milk. Horse milk (horse milk), although not common, is utilized as a milk alternative by nomadic tribes in Central Asia. Meanwhile, "colostrum" is a form of milk produced during the late stage of pregnancy and several days after birth. Unlike regular milk, colostrum contains more nutrients and antibodies necessary for survival and growth, and also compared to regular milk, contains a higher amount of antioxidant materials such as lactoferrin and hemopexin, and is thus sometimes preferred in health improvement and nutritional aspects.

The term "raw milk" as used herein refers to milk collected from a ranch that has not undergone any particular processing such as sterilization, and the term "commercial milk" as used herein refers to milk that is packaged in a commercially available form through a sterilization process and a homogenization process.

The term "skin regeneration" as used herein refers to the process of a skin regeneration cycle, or the process in which in case of a wound, cells are formed in the basal layer, thereby restoring the skin. Epidermal cells formed in the basal layer are gradually pushed to upper layers and arrive at the stratum corneum. This process is referred to as a skin regeneration cycle or a skin turn-over process. Generally, a skin turn-over process is considered to take 28 days, on average, including a period of 14 days in which cells are formed in the basal layer and travel upwards, and a period of another 14 days in which cells remaining on the stratum corneum die and shed off. Since the process of regeneration and shedding takes longer with advancing age, the skin turn-over cycle may take up to 42 days, and it is known that the skin turn-over cycle typically becomes longer after the age of 30. For this reason, skin becomes dull and rough-textured with advancing age, and that is to say, the skin starts to age.

## DETAILED DESCRIPTION OF THE INVENTION

According to one aspect of the present invention, provided is a cosmetic composition for skin regeneration, including exosomes isolated from milk or goat milk as an active ingredient.

In the cosmetic composition for skin regeneration, the milk or goat milk may be raw milk, commercial milk or goat milk, or colostrum.

According to another aspect of the present invention, provided is a cosmetic composition for wrinkle reduction, including exosomes isolated from milk or goat milk as an active ingredient.

In the cosmetic composition for wrinkle reduction, the milk or goat milk may be raw milk, commercial milk or goat milk, or colostrum.

According to another aspect of the present invention, provided is a cosmetic composition for skin whitening, including exosomes isolated from milk or goat milk as an active ingredient.

In the cosmetic composition for skin whitening, the milk or goat milk may be raw milk, commercial milk or goat milk, or colostrum.

The cosmetic composition according to one aspect of the present invention may be used in various products such as a functional cosmetics product, a cleanser, a shampoo, and the like. Examples of products to which the present composition can be added include various types of cosmetics products such as a toner, a lotion, a cream, an essence, a nutrient solution, a cosmetic solution, and a facial mask, and also include soap, shampoo, a hair rinse product, a cleanser, a body cleanser, a facial cleanser, hair treatment, and a beauty solution.

The cosmetic composition of the present invention may further include an additional functional component selected from the group consisting of a water-soluble vitamin, a fat-soluble vitamin, a polymer peptide, a polysaccharide, a sphingolipid, and an algae extract.

The water-soluble vitamin may be any vitamin that can be blended in cosmetics, but may be preferably vitamin B1, vitamin B2, vitamin B6, pyridoxine, pyridoxine hydrochloride, vitamin B12, pantothenic acid, nicotinic acid, nicotinamide, folic acid, vitamin C, vitamin H, and the like, and salts of the aforementioned components (thiamine hydrochloride, sodium ascorbate, etc.) or derivatives thereof (sodium ascorbic acid-2-phosphate, magnesium ascorbic acid-2-phsophate, etc.) are also included in the water-soluble vitamin usable in the present invention. The water-soluble vitamin may be obtained by known methods such as a microbial transformation method, a chemical synthesis method, an enzyme method, or a purification method from microbial cultures, etc.

The fat-soluble vitamin may be any vitamin that can be blended in cosmetics, but may be preferably vitamin A, carotene, vitamin D2, vitamin D3, vitamin E (d1-á-tocopherol, d-á-tocopherol, d-ä-tocopherol) , etc. and derivatives of the aforementioned components (ascorbine palmitate, ascorbine stearate, ascorbine dipalmitate, dl-á-tocopherol acetate, dl-á-tocopherol nicotinic acid, vitamin E, DL-pantothenyl alcohol, D-pantothenyl alcohol, pantothenyl ethyl ether, etc.) are also included in the oil-soluble vitamin usable in the present invention.

The fat-soluble vitamin may be obtained from microbial cultures by known methods such as a microbial transformation method, a chemical synthesis method, an enzyme method, or a purification method.

The polymer peptide may be any polymer peptide that can be blended in cosmetics, but may be preferably collagen, hydrolyzed collagen, gelatin, elastin, hydrolyzed elastin, keratin, and the like. The polymer peptide may be obtained from microbial culture media by known methods such as a chemical synthesis method, an enzyme method, or a purification method, or alternatively, may be purified from natural products such as the skins of pigs, cattle, etc. and fibroin of silkworms, and used.

The polysaccharide may be any polysaccharide that can be blended in cosmetics, but may be preferably hydroxyethyl cellulose, xanthan gum, sodium hyaluronate, chondroitin sulfate, or a salt thereof (sodium salt, etc.). For example, the chondroitin sulfate or a salt thereof, etc. may be purified from a known mammal or fish and used.

The sphingolipid may be any sphingolipid that can be blended in cosmetics, but may be preferably a ceramide, a phytosphingosine, a glycosphingolipid, and the like. The sphingolipid may be purified by a known method from mammals, fish, shellfish, yeast, or plant, etc. or may be obtained by a chemical synthesis method.

The algae extract may be any algae extract that can be blended in cosmetics, but may be preferably a brown algae extract, a red algae extract, a green algae extract, and the like, and carrageenan, alginic acid, sodium alginate, potassium alginate, etc. purified from the aforementioned algae extracts are also included in the algae extract used in the present invention. The algae extract may be obtained from algae through purification by a known method.

In a cosmetic material of the present invention, other ingredients that are commonly blended in cosmetic materials may be blended in together with the above-mentioned essential ingredients.

Other ingredients that may be added include an oil and fat component, a humectant, an emollient, a surfactant, organic and inorganic pigments, organic particles, a UV absorber, a preservative, a disinfectant, an antioxidant, a plant extract, a pH adjuster, an alcohol, a pigment, a fragrance, a blood-circulation promotor, a cooling agent, an adiaphoretic agent, purified water, and the like.

The oil and fat component may include ester-based oil and fat, hydrocarbon-based oil and fat, silicone-based oil and fat, fluorine-based oil and fat, animal oil and fat, vegetable oil and fat, and the like. The ester-based oil and fat may include esters such as glyceryl tri2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linoleate , isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate, tri(capryl, capric acid) glyceryl, tri 2-ethylhexanoate trimethylolpropane, trimethylolpropane triisostearate, tetra 2-ethylhexanoate pentaerythritol, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linoleate, isopropyl isostearate, cetostearyl 2-ethylhexanoate, stearyl 2-ethylhexanoate, hexyl isostearate, ethylene glycol dioctanoate, ethylene glycol dioleate, propylene glycol dicapric acid, di(capryl capric acid) propylene glycol, propylene glycol dicaprylate, neopentyl glycol dicaprylate, neopentyl glycol dioctanoate, glyceryl tricaprylate, glyceryl triundecanoate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerol oleic acid ester, polyglycerol isostearic acid ester, triisocetyl citrate, triisoalkyl citrate, triisooctyl citrate, lauryl lactate, myristyl lactate, cetyl lactate, octyldecyl lactate, triethyl citrate, acetyl triethyl citrate, acetyl tributyl citrate, trioctyl citrate, diisostearyl malate, 2-ethylhexyl hydroxystearate, di2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stealoyl hydroxystearate, stearyl 12-stearoyl hydroxystearate, isostearyl 12-stearoylhydroxystearate, and the like.

The hydrocarbon-based oil and fat may include hydrocarbon-based oil and fat, such as squalene, liquid paraffin, á-olefin oligomer, isoparaffin, ceresin, paraffin, liquid isoparaffin, polybudene, microcrystalline wax, petrolatum, and the like.

The silicone-based oil and fat may include polymethylsilicone, methylphenylsilicone, methylcyclopolysiloxane, octamethylpolysiloxane, decamethylpolysiloxane, dodecamethylcyclosiloxane, dimethylsiloxane/methylcetyloxysiloxane copolymer, dimethylsiloxane/methylstealloxysiloxane copolymer, alkyl-modified silicone oil, amino modified silicone oil, and the like.

The fluorine-based oil and fat may include perfluoropolyether and the like.

The animal or vegetable fats and oils may include animal and vegetable fats and oils such as avocado oil, almond oil, olive oil, sesame seed oil, rice bran oil, saffron oil, soybean oil, corn oil, rapeseed oil, apricot kernel oil, palm kernel oil, palm oil, castor oil, sunflower oil, grape seed oil, cottonseed oil, palm oil, kukui nut oil, wheat germ oil, rice germ oil, shea butter, evening primrose oil, macadamia nut oil, meadowfoam seed oil, egg yolk oil, tallow, horse oil, mink oil, orange roughy oil, jojoba oil, candelabra wax, carnauba wax, liquid lanolin, and hydrogenated castor oil.

The humectant may include a water-soluble low-molecular weight humectant, a fat-soluble molecule humectant, a water-soluble polymer, a fat-soluble polymer, and the like.

The water-soluble low-molecular weight humectant may include serine, glutamine, sorbitol, mannitol, sodium pyrrolidone carboxylate, glycerin, propylene glycol, 1,3-butylene glycol, ethylene glycol, polyethylene glycol (degree of polymerization n = 2 or more), polypropylene glycol (degree of polymerization n = 2 or more), polyglycerin (degree of polymerization n = 2 or more), lactic acid, a lactate, and the like.

The fat-soluble low-molecular weight humectant may include cholesterol, cholesterol esters, and the like.

The water-soluble polymer may include a carboxyvinyl polymer, polyaspartate, tragacanth, xanthan gum, methylcellulose, hydroxymethylcellulose, hydroxyethylcellulose, hydroxypropylcellulose, carboxymethylcellulose, water-soluble chitin, chitosan, dextrin, and the like. The fat-soluble polymer may include a polyvinylpyrrolidone/eicosene copolymer, a polyvinylpyrrolidone/hexadecene copolymer, nitrocellulose, a dextrin fatty acid ester, a high molecular-weight silicone, and the like.

The emollient may include a long-chain acylglutamic acid cholesteryl ester, cholesteryl hydroxystearate, 12-hydroxystearic acid, stearic acid, rosin acid, a lanolin fatty acid cholesteryl ester, and the like.

The surfactant may include a non-ionic surfactant, an anionic surfactant, a cationic surfactant, an amphoteric surfactant, and the like.

The non-ionic surfactant may include a self-emulsifying glycerin monostearate, a propylene glycol fatty acid ester, a glycerin fatty acid ester, a polyglycerin fatty acid ester, a sorbitan fatty acid ester, a POE (polyoxyethylene) sorbitan fatty acid ester, a POE sorbite fatty acid ester, a POE glycerin fatty acid ester, a POE alkyl ether, a POE fatty acid ester, POE hydrogenated castor oil, POE castor oil, a POE/POP (polyoxyethylene/polyoxypropylene) copolymer, a POE/POP alkyl ether, a polyether-modified silicone, a lauric acid alkanolamide, an alkyl amine oxide, a hydrogenated soybean phospholipid, and the like.

The anionic surfactant may include a fatty acid soap, an á-acyl sulfonate, an alkyl sulfonate, an alkyl allyl sulfonate, an alkyl naphthalene sulfonate, an alkyl sulfate, a POE alkyl ether sulfate, an alkyl amide sulfate, an alkyl phosphate, a POE alkyl phosphate, an alkyl amide phosphate, an alkyloyl alkyl taurine salt, an N-acyl amino acid salt, a POE alkyl ether carboxylate salt, an alkyl sulfosuccinic acid salt, sodium alkyl sulfoacetate, an acylated hydrolyzed collagen peptide salt, a perfluoroalkyl phosphate ester, and the like.

The cationic surfactant may include alkyl trimethyl ammonium chloride, stearyl trimethyl ammonium chloride, stearyl trimethyl ammonium bromide, cetostearyl trimethyl ammonium chloride, distearyl dimethyl ammonium chloride, stearyl dimethyl benzyl ammonium chloride, behenyl trimethyl ammonium bromide, benzalkonium chloride, diethyl amino ethyl amide stearate, dimethyl amino propyl amide stearate, a lanolin derivative, a quaternary ammonium salt, and the like.

The amphoteric surfactant may include a carboxybetaine type, an amidebetaine type, a sulfobetaine type, a hydroxysulfobetaine type, an amidesulfobetaine type, a phosphobetaine type, an aminocarboxylate type, an imidazoline derivative type, an amidamine type, and the like.

The organic and inorganic pigments may include inorganic pigments such as silicic acid, silicic anhydride, magnesium silicate, talc, sericite, mica, kaolin, Bengala, clay, bentonite, titanium coated mica, bismuth oxychloride, zirconium oxide, magnesium oxide, zinc oxide, titanium oxide, aluminum oxide, calcium sulfate, sulfate barium, magnesium sulfate, calcium carbonate, magnesium carbonate, iron oxide, ultramarine blue, chromium oxide, chromium hydroxide, calamine, carbon black, and a complex thereof; and organic pigments such as polyamide, polyester, polypropylene, polystyrene, polyurethane, vinyl resin, urea resin, phenol resin, fluororesin, silicon resin, acrylic resin, melamine resin, epoxy resin, polycarbonate resin, a divinylbenzene-styrene copolymer, silk powder, cellulose, CI Pigment Yellow, CI Pigment Orange, and the like; and a composite pigment of such inorganic pigments and organic pigments, and the like.

The organic particles may include metal soaps such as calcium stearate; alkyl phosphate metal salts such as sodium cetylate, zinc laurylate, and calcium laurylate; acyl amino acid polyvalent metal salts such as N-lauroyl-â-alanine calcium, N-lauroyl-â-alanine zinc, and N-lauroylglycine calcium; amidesulfonic acid polyvalent metal salts such as L-lauroyl-taurine calcium and N-palmitoyl-taurine calcium; N-acyl basic amino acids such as Nà-lauroyl-L-lysine, Nà-palmitoylizine, Ná-paritoylolnithine, Ná-lauroylarginine, and Ná-hydrogenated beef fatty acid acylarginine; N-acyl polypeptides such as N-lauroylglycylglycine; á-amino fatty acids such as á-aminocaprylic acid and á-aminolauric acid; and polyethylene, polypropylene, nylon, polymethyl methacrylate, polystyrene, divinylbenzene-styrene copolymers, ethylene tetrafluoride, and the like.

The UV absorber may include para-aminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomentyl salicylate, benzyl cinnamate, 2-ethoxyethyl para-methoxycinnamate, octyl para-methoxycinnamate, mono-2-ethylhexane glyceryl dipara-methoxycinnamate, glyceryl, isopropyl para-methoxycinnamate, a diisopropyl-diisopropyl cinnamic acid ester mixture, urocanic acid, ethyl urokanate, hydroxy methoxy benzophenone, hydroxy methoxy benzophenone sulfonic acid and salts thereof, dihydroxy methoxy benzophenone, sodium dihydroxy methoxy benzophenone disulfonate, dihydroxy benzophenone, tetrahydroxy benzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The disinfectant may include hinokitiol, triclosan, trichlorohydroxy diphenyl ether, chlorhexidine gluconate, phenoxyethanol, resorcin, isopropyl methylphenol, azulene, salicylic acid, zinc pyrithione, benzalkonium chloride, photosensitizer No. 301, sodium mononitro guaiacol, undecylenic acid, and the like.

The antioxidant may include butylhydroxyanisole, propyl gallate, elisorbic acid, and the like.

The pH adjuster may include citric acid, sodium citrate, malic acid, sodium malate, fumaric acid, sodium fumarate, succinic acid, sodium succinate, sodium hydroxide, sodium monohydrogen phosphate, and the like.

The alcohol may include a higher alcohol such as cetyl alcohol.

In addition, ingredients that may be added are not limited to the above. Also, any of the aforementioned ingredients may be added within a range that does not adversely affect the purpose and effect of the present invention, but may be added preferably in an amount of 0.01-5 wt%, more preferably of 0.01-3 wt%, with respect to the total weight.

The cosmetic material of the present invention may take a form of a solution, an emulsion, a viscous mixture, and the like.

Examples of the form of the cosmetic material are not particularly limited but may include emulsion, cream, cosmetic water, a facial mask, a makeup foundation, a lotion, beauty solution, hair cosmetic materials, soap, and the like.

Specific examples of the cosmetic material of the present invention may include facial cleansing cream, facial cleansing foam, cleansing cream, cleansing milk, cleansing lotion, massage cream, cold cream, moisturizer cream, emulsion, cosmetic water, a facial pack, after-saving cream, sun protection cream, tanning oil, soap, body shampoo, hair shampoo, hair rinse, hair treatment, hair conditioner, hair growth material, hair cream, hair liquid, hair-setting lotion, hair spray, hair bridge, color rinse, color spray, a permanent wave solution, pressed powder, loose powder, eye shadow, hand cream, lipstick, and the like.

The cosmetic composition of the present invention may be obtained by preparing ingredients selected from the group consisting of a water-soluble vitamin, a fat-soluble vitamin, a polymer peptide, a polysaccharide, sphingolipids, and an algae extract, (other ingredients that can be added as needed, other than the above-mentioned ingredients) in addition to exosomes isolated from milk or goat milk, which is the active ingredient of the present invention, in accordance with a known method, for example, a method disclosed in ⌜Manual for Percutaneous Application Preparation Developmen⌟

edited by Matsumoto Michio, Edition 1 (published in 1985 by Seishi Shoin), and the like.

According to another aspect of the present invention, provided is a functional cosmetic product for hair for alleviating and preventing hair loss, including exosomes isolated from milk or goat milk as an active ingredient.

The functional cosmetic product for hair for alleviating and preventing hair loss according to one aspect of the present invention may be provided as a shampoo formulation, or may be provided as topical formulations such as ointment, cream, and gel for scalp application.

According to another aspect of the present invention, provided is a functional health food including exosomes isolated from milk or goat milk as an active ingredient.

Health-related functions of the functional health food may be, but are not limited to, alleviation of skin hypersensitivity, improvement of immune functions, improvement of gut health, improvement of skin health, blood sugar level regulation, antioxidant function, or improvement of liver health.

The type of the functional health food according to one aspect of the present invention are not particularly limited. The type of the functional health food may be, without being limited to, any one of the group consisting of dairy products, confectionery products, condiments, beverages and drinks, snacks, candies, ice cream and frozen desserts, breakfast cereals, nutrition bars, snack bar chocolate products, processed foods, cereal products and pasta, soups, sauce and dressings, confectionery products, oils and fat products, dairy drinks and milk drinks, soy dairy-like products, frozen foods, cooked and alternative foods, meat products, cheese, yogurts, breads, rolls, yeast products, cakes, cookies, and crackers. Also, all items that are considered as functional food in a general sense are included.

The functional health food according to one aspect of the present invention may be formulated and used as capsules, tablets, powder, liquid suspension, pills, granules, and the like. Such formulations are formed by forming the functional health food, as is, or evenly mixed with an excipient, a binder, a disintegrant, etc. into particles by an appropriate method and making the particles as even as possible. Further, a flavoring agent, a bitterant, or the like may be added as needed. When the functional health food is a functional health food in a beverage formulation, the functional health food may contain various flavoring agents or natural carbohydrates, etc. as additional ingredients, as is the case with common beverages. Examples of the natural carbohydrates include monosaccharides such as glucose and fructose, disaccharides such as maltose and sucrose, polysaccharides such as dextrin and cyclodextrin, and sugar alcohols such as xylitol, sorbitol, and erythritol. For the sweetener, natural sweeteners such as thaumatin and stevia extract, or artificial sweeteners such as saccharin and aspartame may be used. Functional materials such as a deer antler extract, fructooligosaccharide for helping calcium absorption and bowel movement, acacia honey, a composite Scutellaria baicalensis extract which is a natural preservative, gellan gum, which is an anti-settling thickener, and the like, may be added, but without being limited thereto, any functional material that is suitable for functional health food may be appropriately used.

Exosomes isolated from milk or goat milk, which is the active ingredient of the functional health food of the present invention, are an extremely safe material, in that the source, milk or goat milk, is a food material that already has been used as a food source by the mankind for a long time. Accordingly, the milk- or colostrum-derived exosomes according to an example of the present invention may be used as a safe and extremely efficient source material in various health functions.

According to another aspect of the present invention, provided is a pharmaceutical composition for wound treatment, including exosomes isolated from milk or goat milk as an active ingredient.

In the pharmaceutical composition, the milk or goat milk may be raw milk, commercial milk or goat milk, or colostrum.

In the pharmaceutical composition of the present invention, the active ingredient may be included in an amount of 0.1 to 100 wt% with respect to the total weight of the composition.

The composition of the present invention may further include an appropriate carrier, excipient, and diluent commonly used in the preparation of pharmaceutical compositions. In addition, in the preparation of pharmaceutical compositions, solid or liquid additives for formulation may be used. The additives for formulation may be any one of organic and inorganic.

Examples of the excipient may include lactose, sucrose, saccharose, glucose, corn starch, starch, talc, sorbit, crystalline cellulose, dextrin, kaolin, calcium carbonate, silicon dioxide, and the like. Examples of a binder may include polyvinyl alcohol, polyvinyl ether, ethyl cellulose, methyl cellulose, Arabia rubber, tragacanth, gelatin, shellac, hydroxypropyl cellulose, hydroxypropyl methyl cellulose, calcium citrate, dextrin, pectin, and the like. Examples of a lubricant may include magnesium stearate, talc, polyethylene glycol, silica, hydrogenated vegetable oil, and the like. Any coloring agent that is commonly permitted for use in drugs may be used. Tablets and granules of the pharmaceutical composition may be appropriately coated by sugar coating, gelatin coating, and the like, as needed. In addition, preservatives, an antioxidant, and the like may be added as needed.

The pharmaceutical composition of the present invention may be prepared in any formulation that is commonly produced in the corresponding industry (e.g., literature [Remington's Pharmaceutical Science, new edition; Mack Publishing Company, Easton PA), and the form of formulation is not particularly limited, but may be preferably a topical agent. The topical agent of the present invention may include common forms of topical agent, such as a sheet preparation, a liquid topical agent, a spray preparation, a lotion preparation, a cream preparation, a puff preparation, a powder preparation, a penetration pad preparation, a spray preparation, a gel preparation including a hydrogel, a paste preparation, a liniment preparation, an ointment preparation, aerosol, a powder preparation, a suspension preparation, a transdermal preparation, and the like. Such formulations are described in the literature [Remington's Pharmaceutical Science, 15th Edition, 1975, Mack Publishing Company, Easton, Pennsylvania 18042 (Chapter 87: Blaug, Seymour), which is the formulary generally known in all pharmaceutical and chemical fields.

As an example of the present invention, the composition may be directly applied to a wound. That is, the composition may be distributed on a wound site. A sheet form of the composition, when applied to a wound site, serves to appropriately dress the applied site so as to protect the wound and prevent a decrease in the therapeutic effect of the active ingredient. Any dressings that are commercially available or commonly known may be used. Examples of commercially available dressings may include Compeel, Duoderm, Tagaderm, and Opsite.

When the pharmaceutical composition of the present invention is provided as a topical agent, a pharmaceutically acceptable carrier, although it varies depending on the formulation of the pharmaceutical composition, may include hydrocarbons such as petrolatum, liquid paraffin, and gelled hydrocarbons (known as Plastibase); animal and vegetable oils, such as medium-chain fatty acid triglyceride, pork fat, hard fat, and cacao fat; higher fatty acid alcohols, fatty acids and esters thereof, such as cetanol, stearyl alcohol, stearic acid, and isopropyl palmitate; water-soluble bases such as macrogol (polyethylene glycol), 1,3-butylene glycol, glycerol, gelatin, sucrose, and sugar alcohol; emulsifiers, such as glycerol fatty acid ester, polyoxylstearate, and polyoxyethylene hydrogenated castor oil; adhesives, such as acrylic acid ester and sodium alginate; propellants such as liquefied petroleum gas and carbon dioxide; and preservatives such as paraoxybenzoic acid esters, and the topical agent of the present invention may be manufactured according to a known method, utilizing any of the aforementioned components. Other than the aforementioned components, a stabilizer, a fragrance, a coloring agent, a pH adjuster, a diluent, a surfactant, a preservative, an antioxidant, and the like, may be further blended therein as needed. The topical agent of the present invention may be used by application to a local wound site by a known method.

In addition, the topical agent of the present invention may be used as adhered on a solid support, such as a wound peeling cover of a common bandage. As an example of the present invention, a solid support is first coated with an adhesive layer to increase the attachment of a liquid medium to the solid support. Examples of an adhesive agent may include polyacrylate and cyanoacrylate.

This type of formulation is widely available in the market, examples including: bandages having a non-adhesive wound peeling cover in the form of a perforated plastic film (Smith & Nephew Ltd.), BAND-AID in the form of a thin strip, patch, spot, and plastic strip by Johnson & Johnson; Curity CURAD bandages (Curity CURAD, Ouchless) by Colgate-Palmolive Co.(Kendall); and STIK-TITE elastic strips by American WhiteCross Laboratories, Inc., and the like.

As an example of the present invention, the pharmaceutical composition according to the present invention may be formulated in the form of a liquid topical agent by mixing milk- or goat milk-derived exosomes according to an example of the present invention with saline in a certain volume ratio. As an example of the present invention, the pharmaceutical composition according to the present invention may be formulated in the form of an ointment by mixing the milk- or goat milk-derived exosomes according to an example of the present invention with a water-soluble ointment base and adding saline thereto.

According to another aspect of the present invention, provided is a method of accelerating regeneration of wounded skin of a subject, the method including applying a therapeutically effective amount of the exosomes isolated from milk or goat milk to wounded skin of the subject.

In the method, the milk or goat milk may be raw milk, commercial milk or goat milk, or colostrum.

The therapeutically effective amount may vary depending on the type of a patient's wound, application site, a treatment frequency, a treatment duration, a formulation, the state of the patient, the type of aids, and the like. The amount of use is not particularly limited, but the daily effective dose of the pharmaceutical composition of the present invention may be, when cell culture medium is applied to the wound, 1 to 50 $\mu$l/cm$^2$, preferably 5 to 20 $\mu$l/cm$^2$.

The dose for 1 day may be administered once daily, or may be distributed over two or three administrations daily at an appropriate interval, or may be administered intermittently every few days.

The step of administering may be performed by oral administration, subcutaneous administration, intravenous administration, intramuscular administration, or intranasal administration, but intravenous administration or subcutaneous administration is preferable, and if formulated as an appropriate skin-application type formulation, a direct application to a wound site is also possible.

According to another aspect of the present invention, provided is a method of enhancing immunity of a subject, the method including administering a therapeutically effective amount of exosomes isolated from milk or goat milk to the subject.

In the method, the milk or goat milk may be raw milk, commercial milk or goat milk, or colostrum.

According to another aspect of the present invention, provided is a method of preventing or treating hair loss of a subject, the method including administering a therapeutically effective amount of exosomes isolated from milk or goat milk to the subject.

In the method, the milk or goat milk may be raw milk, commercial milk or goat milk, or colostrum.

The step of administering may be performed by oral administration, subcutaneous administration, intravenous administration, intramuscular administration, or intranasal administration, but intravenous administration or subcutaneous administration is preferable, and if formulated as an appropriate skin-application type formulation, a direct application to a wound site is also possible.

According to another aspect of the present invention, provided is a method of reducing skin wrinkles of a subject, the method including administering a therapeutically effective amount of exosomes isolated from milk or goat milk to the subject.

In the method, the milk or goat milk may be raw milk, commercial milk or goat milk, or colostrum.

The step of administering may be performed by oral administration, subcutaneous administration, intravenous administration, intramuscular administration, or intranasal administration, but intravenous administration or subcutaneous administration is preferable, and if formulated as an appropriate skin-application type formulation, a direct application to a wound site is also possible.

According to another aspect of the present invention, provided is a method of whitening the skin of a subject, the method including administering a therapeutically effective amount of exosomes isolated from milk or goat milk to the subject.

In the method, the milk or goat milk may be raw milk, commercial milk or goat milk, or colostrum.

The step of administering may be performed by oral administration, subcutaneous administration, intravenous administration, intramuscular administration, or intranasal administration, but intravenous administration or subcutaneous administration is preferable, and if formulated as an appropriate skin-application type formulation, a direct application to a wound site is also possible.

According to another aspect of the present invention, provided is a use of exosomes isolated from milk or goat milk for the preparation of a wound healing agent.

According to another aspect of the present invention, provided is a use of exosomes isolated from milk or goat milk for the preparation of cosmetics for wrinkle reduction.

According to another aspect of the present invention, provided is a use of exosomes isolated from milk or goat milk for the preparation of cosmetics for whitening.

According to another aspect of the present invention, provided is a use of exosomes isolated from milk or goat milk for the preparation of an agent for preventing and treating hair loss.

According to another aspect of the present invention, provided is a use of exosomes isolated from milk or goat milk for the preparation of a hair growth promoting·hair growing agent.

According to another aspect of the present invention, provided is a use of exosomes isolated from milk or goat milk for the preparation of a composition for improving immunity.

According to another aspect of the present invention, provided is a method of isolating exosomes from milk or goat milk, the method including: a first centrifugation step of centrifuging milk or goat milk to remove fat and cells therefrom; a strainer filtration step of filtering the centrifuged milk or goat milk with a strainer having a mesh size of 20 to 60 $\mu$m to further remove fat and cells therefrom; a dilution step of diluting the filtered milk or goat milk by adding an equal volume of distilled water thereto; an isoelectric precipitation step of adjusting the diluted milk or goat milk to a pH of 4 to 5 by adding an acid thereto, to precipitate casein in the milk or goat milk; a secondary centrifugation step of additionally centrifuging the milk or goat milk having casein precipitated therein, and collecting a supernatant therefrom; and a filtration step of filtering the collected supernatant by a 0.2 $\mu$m filter.

In the above method, the first centrifugation step may be carried out under a temperature condition of 4°C at 4,000 to 6,000 xg for 20 to 40 minutes, and more preferably, may be carried out under a temperature condition of 4°C at 4,000 to 5,000 xg for 30 minutes.

In the above method, the strainer may be a strainer having a mesh size of 30 to 50 $\mu$m, more preferably may be a strainer having a mesh size of 35 to 45 $\mu$m, and most preferably may be a strainer having a mesh size of 40 $\mu$m.

In the above method, the acid may be hydrochloric acid, nitric acid, acetic acid, or sulfuric acid, more preferably may be hydrochloric acid, and most preferably may be 6N hydrochloric acid.

In the above method, the second centrifugation step may be carried out at room temperature at 4,000 to 6,000 xg for 15 to 30 minutes, more preferably may be carried out at room temperature at 4,500 to 5,500 xg for 15 to 25 minutes, and most preferably may be carried out at room temperature at 5,000 xg for 20 minutes.

It is known that exosomes are abundantly present in milk. Moreover, since milk can serve as an exosome production platform capable of producing exosomes in large quantities at a reduced cost compared to stem cells, a process for producing exosomes in large quantities from milk or colostrum was established (FIG. 1a). Moreover, through the process established above, it could be confirmed that when isolating exosomes from milk, a significantly larger amount of exosomes was isolated compared to when isolating from HEK293 cells (see FIG. 2a), and particularly, it was found that when using colostrum as the raw material, exosomes were produced with an extremely high yield of 0.45 to 1.5 mg/ml when converted to a protein content. It could be seen that this is a value up to 100 times or higher, when compared to the yield of HEK293 cells (see FIG. 2). Moreover, the present inventors, in an attempt to increase exosome production yields from milk, have developed a simpler process that does not utilize ultrafiltration (FIG. 1b), and also confirmed that the use of such an improved process to isolate exosomes from milk is capable of achieving a remarkable yield increase 400 to 1,000 times or more, compared to the conventional ultrafiltration technique (FIG. 2b). Meanwhile, in order to confirm whether the exosomes prepared by the above method are normal exosomes, the expression of exosome markers CD8, CD63, CD81, and TSG 101 was analyzed by Western blot analysis, and the particle size distribution of exosomes was analyzed by dynamic scattering analysis. As a result, as could be seen in FIG. 3, the exosome markers were identified as normal, although slightly lower than the HEK293-derived exosomes serving as the control, and as could be seen in FIG. 4, it was found that the milk-derived exosomes were nano-sized particles having an average particle diameter of 68.05 nm and a particle size distribution of 30 to 100 nm. This is a size distribution similar to that of the control exosomes, and from this result, it could be confirmed that exosomes could be normally isolated from milk.

The present inventors performed stability analysis in order to investigate the utility of exosomes isolated from cell culture media as a raw material for cosmetics. In particular, exosomes isolated from HEK293 cells, which is a human cell line generally used in the production of recombinant exosomes, were freeze-dried and thawed twice, and their shapes were examined by dynamic scattering analysis and a transmission electron microscope (see FIG. 3 and FIG. 4). As a result, it was found that the exosomes derived from HEK293 cells, when freeze-dried and thawed for the second time, were incapable of retaining their original shapes due to aggregation. Accordingly, it could be confirmed that exosomes derived from a general cell line are used in the form of a freeze-dried raw material, and are an unsuitable raw material for ingredients in a cosmetic composition that is intended to be later stored at room temperature.

In this context, the present inventors performed comparative analysis between the stability of exosomes isolated from milk and that of exosomes derived from other cells. In particular, particle size analysis by dynamic light scattering was performed between colostrum-derived exosomes according to an example of the present invention and exosomes derived from other cells (HaCat Exo, B16 Exo, and HDF Exo) before and after cryopreservation. It was found that, unlike the control exosomes, milk-derived exosomes retained their shape intact despite freeze-drying and thawing (FIGS. 7a and 7b ), and colostrum exosomes before and after freezing were administered to human dermal fibroblasts, and as a result, it could be confirmed that the ability to induce type I collagen expression remained intact. This shows that biological activity of the milk exosomes according to an example of the present invention remain intact despite the cryopreservation treatment.

Moreover, while the milk exosomes according to an example of the present invention were found to maintain their size despite repeated freezing and thawing, it was found that the other cell-derived exosomes increase in particle size as the number of freeze-thaw cycles increases, and thus give rise to aggregation or fusion, exhibiting an unstable appearance (see FIG. 8 ).

Furthermore, to confirm whether or not the exosomes isolated from milk can be successfully delivered to skin cells, the present inventors treated human dermal fibroblasts (HDF) with fluorescent-labeled milk-derived exosomes and identified the location of the exosomes by a fluorescence microscope (see FIG. 9). The result shows that the milk-derived exosomes of the present invention were well transitioned to the cytoplasm of dermal fibroblasts.

Meanwhile, to confirm whether milk exosomes according to an example of the present invention are potentially capable of giving rise to cytotoxicity, the present inventors administered the milk exosomes to human dermal fibroblasts at a high concentration of 0.1 mg/ml and 0.3 mg/ml, and the result shows that apoptosis did not occur (see FIG. 10).

In addition, to confirm whether the milk exosome according to an example of the present invention possess inhibitory activity against UV-induced oxidative cell damage, after inducing damage to human dermal fibroblasts by UV light, colostrum exosomes according to an example of the present invention were administered thereto to confirm the formation of reactive oxygen species. The result shows that unlike the control exosomes derived from other cells, the colostrum exosomes according to an example of the present invention significantly inhibited the formation of UV-induced reactive oxygen species (FIGS. 11a and 11b).

Furthermore, the present inventors investigated whether milk-derived exosomes isolated according to an example of the present invention possess skin regeneration capability. To this end, human keratinocyte HaCaT cells and human dermal fibroblasts (HDF) were treated with commercial milk- and colostrum-derived exosomes to examine the degree of cell proliferation (see FIG. 6). The result shows that the milk-derived exosomes of the present invention had superior cell proliferation effect compared to the control group (PBS treated). In particular, it was found that the colostrum-derived exosomes has even higher cell proliferation capability than the commercial milk-derived exosomes. Furthermore, the present inventors investigated the influence on cell migration ability, which is one of the measures of cell regeneration capability (see FIG. 13). The result shows that the milk-derived exosomes of the present invention, when administered, increased cell migration in human dermal fibroblasts (HDF) as well as in human keratinocyte HaCaT cells, compared to the control, and the colostrum-derived exosomes in particular exhibited a significantly high cell migration capability.

Furthermore, to identify various uses of milk exosomes according to an example of the present invention, the present inventors investigated the following: the presence or absence of tube formation when treated in vascular endothelial cells (FIG. 14); analysis of in vivo distribution pattern over time when administered in vivo (FIG. 15a to 15c); comparison of wound healing capacity when administered to a wound artificially introduced in an actual wound model animal (FIG. 16); analysis of elastin expression when treated to a wound site (FIG. 17); a skin wrinkle reduction effect via induction of type I collagen expression and inhibition of MMP-2 expression when treated in human dermal fibroblasts (FIGS. 18a and 18b); a skin whitening effect via inhibition of melanin synthesis in melanoma cells (FIG. 19); and a therapeutic effect on hair loss, through the ability to induce the expression of cytokines associated with immune activation when treated in bone marrow-derived macrophages (BMDM) (FIG. 20), the ability to induce the expression of growth factors associated with inflammation when treated in fibroblasts (FIG. 21), uptake into human dermal papilla cells (FIG. 22a), proliferative capacity on human dermal papilla cells in normal and hair loss-induced conditions (FIG. 22b), and analysis on hair growth promotion and hair growth in an animal experiment (FIG. 22c). As a result, it could be confirmed that milk exosomes according to an example of the present invention are a substance that exhibits various biological activities such as wound healing, wrinkle reduction, whitening effect, immunity enhancing effect, hair loss prevention effect, and the like.

From this result, the present inventors were able to confirm that the milk-derived exosomes according to an example of the present invention can be used very effectively for a wide variety of purposes, including skin regeneration and wound healing, and also in the production of functional cosmetics such as whitening and wrinkle reduction, hair loss treatment and immunity enhancement.

## MODE OF CARRYING OUT THE INVENTION

Hereinbelow, the present invention will be described in greater details through examples and experimental examples. However, the present invention is not limited to the examples and experimental examples disclosed below, but can be implemented in various different forms, and the following examples and experimental examples are provided to make the disclosure of the present invention complete, and to fully inform those of ordinary skill in the art of the scope of the invention.

### Comparative Example: Isolation of Exosomes from HEK293 Cells

In order to isolate exosomes from HEK293 cells, HEK293T cells ($6 \times 106$) were cultured in high-glucose culture media supplemented with 10% FBS and 1% antibiotics (Dulbecco's modified Eagle's medium, DMEM, 4,500 mg/L glucose), maintained at 37°C, 5% $CO_2$ conditions, and when 80-90% confluency was observed on a 15 cm culture dish, and in order to isolate exosomes, cell culture supernatant liquid was obtained by a differential centrifugation method. The specific method is as follows.

First, in order to remove cell debris and other cellular components from the culture medium containing exosomes, centrifugation was sequentially performed at 300 g for 10 minutes, 2,000 g for 10 minutes, and 10,000 g for 30 minutes, and after filtering the culture medium by a 0.22 μm filter, ultra-centrifugation was performed at 36,900 rpm for 2 hours. The exosomes subsequently obtained were resuspended in PBS containing protease inhibitors (Roche).

### Example 1: Isolation of Exosomes from Commercial Milk

The present inventors isolated exosomes from commercial milk by the following method.

First, the commercial milk was subjected to centrifugation at 3,000 g for 30 minutes, and then sequentially subjected to ultra-centrifugation at 12,000 g for 1 hour, at 35,000 g for 1 hour, and then at 70,000 g for 3 hours. Then, the resulting product was subjected to a first filtration by a 0.8 μm filter, and a second filtration by a 0.45 μm, and then a third filtration by a 0.2 μm filter. The filtrate was subjected to ultra-centrifugation at 100,000 g for 1 hour, and pellets were collected and suspended in PBS containing protease inhibitors (Roche) (FIG. 1). For exosomes isolated from the milk above, protein concentrations of the isolated exosomes were measured using the BCA protein analysis kit (Bio-Rad) as described for Comparative Examples above.

### Example 2: Isolation of Exosomes from Colostrum

The present inventors isolated exosomes from colostrum by the same method as described in Example 1 above, except that colostrum (obtained from Lee Sung Young Chang-buk Ranch located at 20, Bongsangwandong 1-ro, Gyeseon-myeon, Changnyeong-gun, Gyeongsangnam-do) was used instead of commercial milk.

## Example 3: Improvement of Exosome Isolation Process

The method used in Examples 1 and 2 requires ultracentrifugation and performs sequential centrifugations several times, and thus has a disadvantage that it is time-consuming. In this context, the present inventors sought to develop a simpler and more efficient exosome isolation method.

To this end, in particular, the present inventors have conceived a novel exosome isolation method through modifications to the previously reported method of isolating exosomes from milk (Yamauchi et al., Drug Dev. Ind. Pharm. 45(3): 359-364, 2019). The method reported by Yamauchi et al., above, utilizes the processes of performing centrifugation of raw milk at 2,000 ×g at 4°C for 20 minutes to remove fat and cells (primary centrifugation), performing dilution by adding an equal volume of distilled water (distilled water dilution), performing titration to pH 4.6 by adding 6N HCl, thereby removing casein through isoelectric precipitation (isoelectric precipitation), performing centrifugation at 5,000 ×g at room temperature for 20 minutes (second centrifugation), and performing filtration sequentially, using 1.0, 0.45, and 0.2 μm filters (sequential filtration). The present inventors have made to the above method the modifications to carry out the first centrifugation process at 5,000 xg at 4°C for 30 minutes, introduce a process of further removing fat and cells by prefiltering with a 40 μm strainer (Falcon ®, Corning, USA) prior to dilution with distilled water, and after the second centrifugation, utilize a one-time filtration process using a 0.2 μm filter instead of performing the sequential filtrations.

## Experimental Example 1: Analysis of Exosome Isolation Yields

Yields of the HEK293-derived exosomes isolated according to Comparative Example above, and exosomes respectively isolated from commercial milk and colostrum according to Examples 1 and 2 of the present invention were analyzed using the BCA protein assay kit (Bio-Rad).

As a result, as could be seen in FIG. 2a, it was found that the exosomes isolated from HEK293 cells showed an extremely low yield, whereas according to Example 1 of the present invention, an isolation yield of the commercial milk-derived exosomes was 0.05 mg/ml, which is about 5 times higher than the isolation yield of HEK293-derived exosomes of 0.01 mg/ml. In particular, the case of colostrum showed a yield of 0.45 mg/ml at the least, based on proteins, which is significantly high even when compared to the case of commercial milk.

Meanwhile, as a result of isolating exosomes from milk using the method of Example 3, as could be seen in FIG. 2b, it could be confirmed that using the non-ultrafiltration exosome isolation method according to an example of the present invention results in a significantly higher yield compared to when using a ultrafiltration method. In particular, the colostrum exosomes exhibited a yield of 1,000 times or more compared to the ultrafiltration method. This result indicates that the method of isolating exosomes from milk according to an example of the present invention is a highly desirable method in terms of productivity.

## Experimental Example 2: Identification of Exosome Markers

In order to confirm whether the exosomes isolated from commercial milk and colostrum retained intact characteristics of exosomes, the present inventors performed a Western blot analysis using specific antibodies on exosome markers, CD9, CD63, CD81, and TSG 101.

As a result, as could be seen in FIG. 3, the exosome-specific markers were identified to be normal in the commercial milk- or colostrum-derived exosomes according to an example of the present invention.

## Experimental Example 3: Characterization of Size and Shape of Exosomes

Subsequently, the present inventors analyzed the particle size of exosomes isolated according to an example of the present invention by using a dynamic light scattering (DLS) analysis device (Malvern zetasizer nano ZS, UK), and photographed the produced exosomes by a transmission electron microscope (FIG. 4). As a result, as shown in FIG. 4, it was found that the milk-derived exosomes prepared according to an example of the present invention has a notably narrow spectrum of size around 68.05 nm, which is similar to the control exosomes, and also in terms of the shape, are not significantly different from the control exosomes.

## Experimental Example 4: Analysis of Freeze/Thaw Stability

Intending to utilize the commercial milk- and colostrum-derived exosomes respectively isolated in Examples 1 and 2as a raw material for cosmetics, the present inventors evaluated storage stability, in particular, freeze-thaw stability.

To this end, the control exosomes and the commercial milk-derived exosomes isolated in Example 1 were frozen at - 85°C and thawed at room temperature, and the thawed exosomes were imaged by a transmission electron microscope, and then the thawed exosomes were again frozen at -85°C and thawed at room temperature for a second time, and imaged by a transmission electron microscope (FIG. 5), and particle size distributions were analyzed through dynamic light scattering (FIG. 6) .

As a result, as could be seen in FIG. 5, it was found that while the control exosomes lose the original exosomal shapes as aggregations among exosomes already occurred when thawed after freezing for the first time, the milk-derived exosomes isolated according to an example of the present invention retain their original shapes even when frozen/thawed for the second time.

Moreover, to confirm whether the above difference could be attributed to the cell type of the control exosomes, the present inventors performed a comparative analysis with a wider range of controls. To this end, in particular, the present inventors measured, using dynamic light scattering, the size of particles of colostrum exosomes prepared in Example 2, and exosomes extracted from human keratinocytes (HaCaT), mouse melanoma cells (B16), and human dermal fibroblasts (HDF) before and after freeze-drying (Fig. 7a). As a result, as can be seen in Figure 7a, it was found that, while there is change in particle size in the exosomes derived from three types of cells before and after freeze-drying, the colostrum exosomes according to an example of the present invention shows little change in particle size. In addition, the shape of colostrum exosomes according to an example of the present invention before and after freeze-drying was identified with a transmission electron microscope, and as could be seen in FIG. 7b, there was no significant change in terms of shape before and after the freeze-drying. From here, it could be seen that the colostrum exosomes have excellent structural stability during freeze-drying. Additionally, the present inventors treated human dermal fibroblasts with the colostrum exosomes according to an example of the present invention of before and after freeze-drying at a concentration of 0.1 mg/ml, and after 24 hours, identified the expression level of type 1 collagen protein through staining by immunofluorescence and with a fluorescence microscope (FIG. 7c). As could be seen in FIG. 7C, it was found that collagen synthesis ability was maintained to a certain extent even when thawed after freeze-drying, indicating that the colostrum exosomes also have functional stability during freeze-drying.

Moreover, to confirm freeze-thaw structural stability of milk exosomes, the present inventors compared particle sizes through freeze-thaw cycles (FIG. 8). Using dynamic light scattering analysis, particle sizes of exosomes extracted from colostrum, and exosomes extracted from human keratinocytes (HaCat), mouse melanoma cells (B16), and human dermal fibroblasts (HDF) were measured with an increasing number of freeze-thaw cycles. It was found that the cell exosomes increase in particle size as the number of freeze-thaw cycles increases, while the colostrum exosomes show little change in particle size after up to 5 freeze-thaw cycles. From here, it could be seen that the colostrum exosomes have excellent structural stability during freeze-thaw.

## Experimental Example 5: Evaluation on Delivery Capability into Skin Cells

The present inventors investigated whether milk-derived exosomes isolated according to an example of the present invention are successfully delivered into skin cells.

In particular, after tinting the plasma membrane of the milk-derived exosomes isolated according to an example of the present invention with red fluorescent dye NHS-Cy5.5, the exosomes were administered to human dermal fibroblasts HDF cells, and after 30 minutes, fluorescent signals were observed by a confocal fluorescent microscope. DAPI was used for nuclear counterstaining (FIG. 9).

As a result, as could be seen in FIG. 9, it was found that the milk-derived exosomes according to an example of the present invention are well absorbed into the cytoplasm of dermal fibroblasts.

## Experimental Example 6: Cytotoxicity Evaluation

The present inventors sought to evaluate cytotoxicity of colostrum exosomes by calculating a necrosis/apoptosis ratio in HDF, human dermal fibroblasts, treated with the colostrum exosomes. 24 hours after administrations of the colostrum exosomes at concentrations of 0, 0.1, and 0.3 mg/ml, the human dermal fibroblasts HDF were stained with propidium iodide (PI), which stains the DNA of necrotic cells and shows fluorescence, and with Annexin V/FITC, which reacts with phosphatidyliserines in apoptotic cells and shows fluorescence, and were analyzed with a flow cytometer. As a result, as could be seen in FIG. 10, it was found that the colostrum exosomes showed no cytotoxicity up to a concentration of 0.3 mg/ml, given the ratio of necrotic or apoptotic cells observed compared to the control. This indicates that the colostrum exosomes according to an example of the present invention are safe substances.

## Experimental Example 7: Analysis of antioxidant effect in UV-damaged cells

The present inventors sought to confirm the antioxidant effect of milk exosomes through a decrease in reactive oxygen species that occurs when colostrum exosomes are administered to human keratinocytes, HaCaT, damaged by UV irradiation. 24 hours after treating HaCaT with commercial milk-derived exosomes and colostrum-derived exosomes at a concentration of 0.05 mg/ml, damage was induced to the cells by UV radiation. After 6 hours, using DCF-DA, which reacts with reactive oxygen species and shows fluorescence, the level of reactive oxygen species generated in cells was analyzed through a fluorescence microscope. As a result, as could be seen in FIGS. 11a and 11b, both the commercial milk-derived exosomes and colostrum exosomes showed a significant antioxidant effect in the UV-damaged cells, and it was confirmed that the antioxidant activity was superior to that of stem cell-derived exosomes.

**Experimental Example 8: Evaluation of skin cell regeneration and wound healing efficacy**

Subsequently, the present inventors investigated whether milk-derived exosomes isolated according to an example of the present invention have skin cell regeneration capability.

8-1: **Evaluation of Skin Cell Proliferation Capability**

In particular, HaCaT human keratinocytes, which is a type of skin cell, and HDF used in Experimental Example 5 above were seeded at a concentration of $1 \times 10^4$ cells per well, and treated with commercial milk-derived exosomes or colostrum-derived exosomes at 0.2 mg/ml, and after 48 hours, cells were counted to measure the degree of cell proliferation. The control was treated only with an equal amount of saline (FIGS. 12a and 12b).
As could be seen in FIGS. 12a and 12b, the result shows that the milk-derived exosomes according to an example of the present invention had a significant increase in proliferation capability in both of the skin cells, in comparison to the control. In particular, it was found that exosomes derived from colostrum show a further excellent skin cell proliferation capability than the commercial milk-derived exosomes.

**8-2: Evaluation of Migration of Skin Cells**

In the process of skin regeneration, migration of skin cells is known to play the most important role. Therefore, the present inventors performed a scratch wound assay in order to identify the influence that milk-derived exosomes isolated according to an example of the present invention have on cell migration.
In particular, the HaCaT human keratinocyte cells and HDF were cultured until 80% confluency was reached in a 100 mm dish at the time of the 7th subculture. The center of well in the 24-well plate was marked and 0.1% gelatin solution was added. The plate was incubated at 37°C for 2 hours and rinsed with DPBS once, to prepare a gelatin-coated 24-well plate. Cells were isolated using 0.25% trypsin-EDTA and seeded in the coated plate at a concentration of $9 \times 10^4$ cells per well. The cells were cultured at 37°C overnight, and then a scratch was made to the cells using a 200 $\mu$L pipette tip, and the wells were rinsed twice with DPBS in order to remove debris. Subsequently, 1 mL of 0.5% FBS-containing media was added to each well, and an image was captured by a microscope (Leica, Wetzlar, Germany) to measure scratch time 0h. Next, except the following groups [positive control (10% FBS DMEM 1mL, negative control (0.5% FBS DMEM 1mL), EGF (10 ng/mL, EGF 0.5% FBS DMEM 1mL)], 800 $\mu$L of media containing 0.5% FBS was replaced in each well. Exosomes isolated respectively in Examples 1 and 2 were placed in a transwell insert, and 200 $\mu$L of media containing 1% FBS in DMEM were added to the insert. After culturing the cells at 37°C for 8 hours, the scratched parts were imaged. Scratch width was measured using Image-Pro Plus software (Media Cybernetics, USA) and calculated according to the Equation 1 below.

$$[\text{Equation 1}]$$

$$\text{Relative Wound Area} = \{(A0 - At)/A0\}/\text{Result value of negative control}$$

(In the equation, A0 is an original wound area, and At is a wound area after 48 hours.)
As a result, as could be seen in FIG. 11, the milk-derived exosomes according to an example of the present invention increased migration of skin cells as compared to the control groups. In particular, it was found that exosomes derived from colostrum significantly increase migration of skin cells as compared to milk-derived exosomes. However, since commercial milk may be more advantageous in terms of material supply and cost, these findings do not undermine the utility of exosomes derived from commercial milk as a raw material for functional cosmetics.

**8-3: Analysis of tube formation rate by vascular endothelial cells**

The present inventors treated mouse-derived endothelial cells (SVEC4-10) with colostrum-, commercial milk-, and serum-derived exosomes at a concentration of 200 $\mu$g/ml, and compared the degree of vessel formation between each group.

First, to allow tube formation in the endothelial cells, Matrigel (Corning #356237) serving as an extracellular matrix was pipetted into a 96-well plate by 50 $\mu$L and was allowed to harden at 37°C for about 30 minutes. Subsequently, exosome-treated cells were attached thereon and cultured for about 8 hours. Here, the degree of tube formation was observed and compared using microscope (DIC) images, branch points at which three or more tubes meet, and tube lengths were digitized and shown in graphs (FIG. 14).

As a result of measurement, as could be seen in FIG. 14, the groups treated with milk-derived exosomes, such as commercial milk and colostrum, showed a higher degree of tube formation compared to the untreated control group, and the serum-derived exosome group showed a result that is not remarkably different from and similar to the control group. From here, it could be confirmed that the milk-derived exosomes according to an example of the present invention accelerate blood vessel formation and as such, can be advantageously used for wound healing.

### 8-4: Confirmation of in vivo distribution of milk exosomes through in vivo imaging

The present inventors analyzed in vivo distribution of administered colostrum exosomes through in vivo imaging (IVIS) of mice.

Colostrum exosomes were adjusted to a concentration of 1 $\mu$g/$\mu$l using PBS buffer, and 1 $\mu$l of Flamma 675 NHS ester (BioActs #PWS1515) was added per 100 $\mu$l of exosomes and labeled at 4°C overnight. Thereafter, dye materials unattached to the exosomes were removed by performing an air fuse twice every hour. 100 $\mu$g of exosomes per one mouse was subcutaneously injected into the upper left thigh of a mouse, and in vivo imaging was performed using the IVIS $^\circledR$ in vivo imaging system (PerkinElmer, USA) at the same time every day for 7 days from the day of injection (FIG. 15a). Here, on the second day, five organs and skin tissues were extracted and the degree to which the colostrum exosomes are distributed was identified for each organ (FIGS. 15b and 15c ). As a result, as could be seen in FIG. 15a, it was found that most of the colostrum exosomes leave the body after 3 days, and it took about 7 days to completely leave the body. As a result of organ harvesting, as shown in FIGS. 15B and 15C , the highest intensity of fluorescence was detected in the skin, followed by the livers, the lungs, and the kidneys in that order. From this result, it could be also confirmed that the colostrum exosomes, after remaining in the body, consequently exit the body through the kidneys.

### 8-5: Efficacy analysis using wound model animals

The present inventors proceeded with wound animal modeling to confirm the actual skin tissue regeneration effect of milk-derived exosomes.

As animals, male Balb/c 7-week-old mice were used and wounds were induced on the back using an 8 mm diameter skin biopsy punch. The experimental groups were divided into a total of 5 types (control, serum, commercial milk, colostrum, and colostrum-4 days), and based on the in vivo distribution results of Experimental Examples 8-4, exosomes were administered by subcutaneous injection at a 3-day interval. Except for the colostrum-4 days group, the remaining four groups were administered with the respective substances on Days 1, 4, and 7, and the cholostrum-4 days group was administered with colostrum exosomes on Days 4, 7, and 10. Here, the concentration of a substance administered was 1 $\mu$g/$\mu$l, and the total volume of 100 $\mu$l was administered per animal, and the progress was observed over about 25 days (FIG. 16).

. As a result, as could be seen in FIG. 16, the skin wounds were significantly rapidly regenerated in the group administered with the colostrum exosomes and the colostrum-4 days group, and in particular, the colostrum-4 day group showed a faster regeneration rate than the other groups between Day 4 and Day 10. Following the colostrum-treated group, the skin was regenerated in the order of the group treated with commercial milk exosomes, the control group treated with PBS, and the group treated with serum-derived exosomes, and from here, it was confirmed that the milk-derived exosomes have a higher skin regeneration effect than the other experimental groups.

### 8-6: Immunohistochemical analysis

After the wound model animal experiments, the present inventors performed an immunohistochemical method for tissue analysis.

In particular, after sacrificing the mice on the 25th day of the animal experiment, wound site tissues were excised and left in a fixative solution overnight to fix the tissues. In order to infiltrate the tissues with paraffin, the dehydration process was carried out sequentially from xylene to 100%, 90%, 80%, and 70% ethanol, followed by embedding with paraffin to form a block. Using a tissue slicer, a 6 $\mu$m-thick paraffin tissue section was prepared and tested according to the experiment method provided by the DAB kit (Abcam #ab64264) . First, after removing the paraffin component from the tissues on the slides and inducing a reaction with a hydrogen peroxidase blocking buffer for 10 minutes at room temperature, the slides were boiled at 95°C for 10 minutes in a retrieval solution to break cross-linking between proteins. Then, after inducing a reaction at room temperature for 10 minutes with a protein blocking buffer, the slides were treated with primary anti-elastin antibody (santacruz #sc-58756) and allowed to react at 4°C overnight. On the next day, a biotinylated goat anti-primary antibody solution and a streptavidin-peroxidase solution were added and allowed to react for 10 minutes at room temperature, and DAB chromogen and DAB substrate were mixed in a ratio of 1:50 and placed on antibody-treated tissue slices, and observed for a color change for 10 minutes at room temperature. Finally, counterstaining was performed to stain the nucleus, and the slides were mounted with toluene.

As a result of microscopic examination of the tissues, as could be seen in FIG. 17, it was found that, compared to other groups, a darker brown color was expressed in the tissues of the group treated with the colostrum exosomes in particular, among the milk-derived exosomes, and this may conclusively indicate that the elastin component was most detected in the tissues of this particular group. In addition, such elastin expression enhancing effect of the milk exosomes is closely related to the wrinkle reduction effect described below.

**Experimental Example 9: Analysis of Wrinkle Reduction Effect**

Collagen, which is the main component of extracellular matrix of the dermis, is normally regenerated by collagen-forming enzymes after being broken down during the skin regeneration cycle, and formation of skin wrinkles is a symptom that manifests due to unsuccessful collagen biosynthesis which fails to restore the extracellular matrix of the dermis. In this context, the present inventors investigated by measuring the degree of inhibition of the expression of collagenase, metalloproteinase 2 (MMP-2), and enhancement of collagen biosynthesis, as a measure of skin wrinkle reduction.

To this end, in particular, the present inventors had human skin fibroblasts (HDF) seeded in a 35δ glass-bottom dish ($3 \times 10^5$ cells) and cultured for 24 hours in an incubator of 37°C and 5% $CO_2$. Then, 24 hours after treatment with the commercial milk-derived exosomes and colostrum-derived exosomes isolated in Examples 1 and 2 at a concentration of 0.1 mg/ml, respectively, the collagen type 1 protein was stained using an immunofluorescence method and examined by a fluorescence microscope, and the protein expression level of metalloproteinase 2 was measured by Western blot. As a result, as could be seen in FIGS. 18a and 18b, it was found that the expression level of collagen type 1 protein increased and the expression level of MMP-2 protein decreased in the HDF treated with the commercial milk-derived exosomes and colostrum exosomes, and this finding, taken together with the results of Experimental Example 8-6, shows that the milk exosomes have an excellent skin wrinkle reduction effect.

**Experimental Example 10: Analysis of Whitening Activity**

The present inventors investigated melanogenesis inhibition, which is a typical indicator of whitening activity, to confirm whether milk-derived exosomes according to an example of the present invention possess skin whitening activity.

In particular, the present inventors sought to confirm the melanin pigment reduction effect by measuring the amount of melanin pigment being produced in B16F10, mouse melanoma cells, after treated with milk exosomes (commercial milk-derived exosomes and colostrum exosomes). As a control group, exosomes isolated from B16F10 cells were used. B16F10 cells were treated with the respective exosomes at a concentration of 0, 0.002, 0.005, 0.01, and 0.02 mg/ml, and after 24 hours, the cells were stimulated by UV radiation. Then, after 24 hours, melanin pigment was extracted from an equal amount of cells, and the extracted melanin pigment was measured and analyzed at a wavelength of 490 nm. As a result, as could be seen in FIG. 19, compared to the control group, there was no significant difference in the amount of melanin pigment produced up to a colostrum exosome concentration of 0.002 mg/ml , whereas at treatment concentrations of 0.005 mg/ml to 0.02 mg/ml of colostrum exosomes, the melanin pigment reduction effect was observed. Additionally, B16F10 were treated with exosomes extracted from B16F10, commercial milk-derived exosomes, and colostrum exosomes at a concentration of 0.1 mg/ml to compare the amount of melanin production after 24 hours (C in FIG. 19) . As a result, the largest decrease in the amount of melanin production was observed in the cells treated with the milk exosomes both when the cells were stimulated with UV rays and when no stimulation was applied.

**Experimental Example 11: Analysis of Immune Function Activity**

The present inventors analyzed cytokines in cell culture medium after treating BMDM (bone marrow-derived macrophages) with 50 μg/ml of commercial milk-derived exosomes and colostrum exosomes.

In particular, the present inventors used 1 ml of the cell culture medium to analyze cytokines in the cell culture medium, and utilized the experiment method provided in the cytokine array kit (ARY006). First, a membrane to which primary antibodies against various cytokines are bound was blocked for 30 minutes at room temperature with a blocking buffer, and then 1 ml of the cell culture medium was administered to the membrane and allowed to react at 4°C overnight.

Thereafter, the membrane was treated with a biotin-conjugated secondary-antibody cocktail and allowed to react at 4°C overnight, followed by reaction with HRP-streptavidin at room temperature for 2 hours, and the results thereof were visualized by chemiluminescence (Bio-Rad). As a result, as could be seen in FIG. 19, in the groups treated with commercial milk and colostrum, the expression of CD54, CXCL1, CCL3, CCL5, and TNFá, which are factors involved in T cell and dendritic cell activation and inflammatory responses, was found to be relatively high compared to the untreated control group. This result indicates that the milk exosomes according to an example of the present invention activates immunity.

**Experimental Example 12: Analysis of Capability of Enhancing the Expression of Growth Factors**

The present inventors treated NIH-3T3 fibroblasts with commercially available oil-derived exosomes and colostrum exosomes at a concentration of 100 $\mu$g/ml, respectively, and after 24 hours, analyzed the expression levels of various growth factors from the cell culture medium and cell lysates. For the experiment, 500 $\mu$g of cell lysates was prepared based on an equal amount of proteins, and cell culture medium was added until the total volume of the sample solution reached 1 ml. Analysis of protein expression levels was performed according to the experiment method provided in the growth factor array kit (RayBiotech #AAM-GF-3-4). First, a membrane to which primary antibodies against growth factors are bound was treated with a blocking buffer at room temperature for 30 minutes, and 1 ml of the prepared sample was added thereto and was allowed to react at 4°C overnight. On the next day, the membrane was sufficiently rinsed and then treated with a biotinylated secondary antibody cocktail and allowed to react at room temperature for 2 hours, followed by a reaction with HRP-streptavidin at room temperature for 2 hours, and the result thereof was visualized by chemiluminescence (Bio-Rad). As a result, as could be seen in FIG. 21, in the experimental groups treated with the commercial milk-derived exosomes and colostrum exosomes, HGF, IGF-1, PDGF-AA, and VEGF-A, which are involved in the anti-inflammatory response and tissue regeneration or angiogenesis, were expressed at a higher level compared to in the control group.

**Experimental Example 13: Analysis of proliferation of liver cells**

The present inventors investigated the activity of human-derived liver cells in order to evaluate efficacy of milk-derived exosomes according to an example of the present invention as a raw material for a functional health food for improving liver health.

The human-derived liver cells were seeded in a 96-well plate at a concentration of $1 \times 10^4$ cells per well, and cultured for 24 hours in an incubator of 37°C and a $CO_2$ concentration of 5%. (0.2) mg/ml of commercial milk- and colostrum-derived exosomes, respectively isolated in Examples 1 and 2, were added to and cultured in serum-free DMEM media for 24 hours, and proliferation of cells was evaluated using CCK-8 assay (Dojindo molecular technologies, Japan). The control was only treated with an equal amount of saline. 10 ul of CCK-8 assay solution was added to each well, and after culturing for 4 hours, absorbance values at 450 nm were measured and compared.

**Experimental Example 14: Analysis of Hair loss prevention and hair growth-promoting and hair growth effects**

**14-1: Analysis of milk exosome uptake by dermal papilla cells**

For the milk exosomes according to an example of the present invention to be able to exhibit a preventative or therapeutic effect on hair loss, the milk exosomes need to exhibit a dermal papilla cell proliferative activity, which is to be taken up by dermal papilla cells, which are cells that help hair growth, and promote proliferation. In this context, the present inventors investigated whether milk exosomes according to an example of the present invention are readily taken up by dermal papilla cells in culture.

In particular, the present inventors treated human dermal papilla cells in culture with 100 $\mu$g/ml of commercial milk-derived exosomes labeled with Cy5.5 fluorescent dye for 1 hour, 4 hours, 12 hours and 24 hours, respectively, and the degree of cellular uptake was observed with a fluorescence microscope (FIG. 22a). As a result, as could be seen in FIG. 22a, it was confirmed that the milk exosomes according to an example of the present invention are readily taken up by human dermal papilla cells in proportion to the treatment duration.

**14-2: Analysis of dermal papilla cell proliferation**

The present inventors investigated the influence that milk-derived exosomes according to an example of the present invention have on the proliferation of human dermal papilla cells, to evaluate the effect of improving hair health and alleviating hair loss.

To this end, in particular, the present inventors first treated human dermal papilla cells with 100, 250, and 500 $\mu$g/ml of the commercial milk-derived exosomes prepared in Example 1 for 48 hours, and cells were counted utilizing the experiment method provided in Cell Counting Kit-8 (CK04-20). 20 $\mu$l of the reaction solution of the Cell Counting Kit was added to 200 $\mu$l of cell culture medium, and was allowed to react for 30 minutes at 37°C and 5% carbon dioxide conditions. Thereafter, measurements were made at a wavelength of 450 nm and were visualized. In addition, human dermal papilla cells were with 100 $\mu$M of hair loss-inducing substance (dihydrotestosterone, DHT) for 24 hours, and then treated with 500 $\mu$g/ml of commercial milk-derived exosomes for 48 hours, and then 20 $\mu$l of the reaction solution of the Cell Counting Kit was added to 200 $\mu$l of cell culture solution and allowed to react for 30 minutes at 37°C and 5% carbon dioxide conditions. Thereafter, measurements were made at a wavelength of 450 nm and were visualized (FIG. 22b). As a result, as could be seen in FIG. 22b, it was found that the group treated with 500 $\mu$g/ml of commercial milk-derived exosomes actively induced a cell proliferation, compared to the untreated control group. Meanwhile, it was found that the group treated with only the hair loss-inducing substance showed a decreased cell proliferation activity compared to the control group, but the cell proliferation activity returns to the level before DHT treatment in the group treated with 500 $\mu$g/ml of commercial milk-derived exosomes after treatment with the hair loss-inducing substance. This suggests that the milk exosomes according to an example of the present invention are also effective in preventing hair loss.

**14-3: Analysis of hair growth-promoting and hair growing effect**

Based on the results of Experimental Example 14-2 above, the present inventors investigated the hair growth-promoting and hair-growing effect utilizing a test animal to confirm whether the milk exosomes according to an example of the present invention are effective at promoting hair growth and growing hair in addition to prevention of hair loss.

In particular, the present inventors shaved the entire back area of male C57BL/6 7-week-old mice short and applied a Veet cream and wiped the remaining hair clean with Kimtech (Day 0), and from Day 1, every other day, 20 $\mu$l of the commercial milk-derived exosomes was administered by intradermal injection, in a total of 100 $\mu$l (200 $\mu$g), to 5 sites in the middle of the back (along the spine). Then, the appearance of hair growth was monitored every 3-4 days. As a result, as could be seen in FIG. 22c, it was found that the hair grows at a faster rate in the group administered with the commercial milk-derived exosomes according to an example of the present invention.

Therefore, it could be confirmed that the milk exosomes according to an example of the present invention are also highly effective in promoting hair growth and growing hair, as well as preventing hair loss.

**Preparation Example 1: Lotion**

Lotion containing exosomes derived from commercial milk or colostrum obtained according to an example of the present invention was prepared by mixing in a composition ratio shown in Table 1 below.

**Table 1**

| Mixing ratio of lotion | |
| --- | --- |
| Ingredients | Content (Unit: Wt%) |
| Milk-derived exosomes | 0.5 |
| Glyceryl stearate SE | 1.5 |
| Cetearyl alcohol | 1.5 |
| Lanolin | 1.5 |
| Polysorbate 60 | 1.3 |
| Sorbitan stearate | 0.5 |
| Hydrogenated palm oil | 4.0 |
| Mineral oil | 5.0 |
| Trioctanoin | 2.0 |
| Dimethicone | 0.8 |
| Tocopherol acetate | 0.5 |
| Carboxy vinyl polymer | 0.12 |
| Glycerin | 5.0 |
| 1,3-butylene glycol | 3.0 |

(continued)

| Mixing ratio of lotion | |
|---|---|
| Ingredients | Content (Unit: Wt%) |
| Sodium hyaluronate | 5.0 |
| Triethanolamine | 0.12 |
| Uniside-U 13 | 0.02 |
| Distilled water | Remainder |
| Total | 100 |

**Preparation Example 2: Nutrient Cream**

Nutrient cream containing commercial milk- or colostrum-derived exosomes obtained according to an example of the present invention was prepared by mixing ingredients in a ratio shown in Table 2 below.

**Table 2**

| Mixing ratio of nutrient cream | |
|---|---|
| Ingredients | Content (Unit: Wt%) |
| Milk-derived exosomes | 0.5 |
| Lipophilic glyceryl monostearate | 1.5 |
| Cetearyl alcohol | 1.5 |
| Stearic acid | 1.0 |
| Polysorbate 60 | 1.5 |
| Sorbitan stearate | 0.6 |
| Isostearyl isostearate | 5.0 |
| Squalene | 5.0 |
| Mineral oil | 35.0 |
| Dimethicone | 0.5 |
| Hydroxyethyl cellulose | 0.12 |
| Triethanolamine | 0.7 |
| Glycerin | 5.0 |
| Uniside-U 13 | 0.02 |
| Distilled water | Remainder |
| Total | 100 |

**Preparation Example 3: Preparation of Shampoo for Alleviating Hair Loss**

Shampoo for alleviating hair loss, containing exosomes derived from colostrum or commercial milk obtained according to one example of the present invention, was prepared by mixing ingredients in a mixing ratio shown in Table 3 below.

**Table 3**

| Mixing ratio of shampoo for alleviating hair loss | |
|---|---|
| Ingredients | Content (Unit: Wt%) |
| Milk-derived exosomes | 1.0 |

(continued)

| Mixing ratio of shampoo for alleviating hair loss | |
|---|---|
| Ingredients | Content (Unit: Wt%) |
| Sodium laureth sulfate | 0.7 |
| Sodium lauryl sulfate | 0.7 |
| Polyquaternium-7 | 1 |
| Hydrolyzed silk | 0.5 |
| Panthenol | 1.0 |
| Glycerin | 2.5 |
| Cocamidopropyl betaine | 0.7 |
| Polyquaternium-10 | 0.5 |
| Ethylhexylglycerin | 3.5 |
| Disodium EDTA | 1.0 |
| Citric acid | 0.7 |
| Butylene glycol | 0.7 |
| Phenoxyethanol | 0.5 |
| Fragrance | 0.2 |
| Distilled water | Remainder |
| Total | 100 |

### Preparation Example 4: Preparation of Functional Health Food (Soft Capsule Preparation)

A soft-capsule preparation containing exosomes derived from colostrum or commercial milk obtained according to one example of the present invention was prepared by mixing in a mixing ratio shown in Table 4 below. Ingredients in the following amounts were mixed and homogenized and then were filled in a soft capsule of a predetermined weight according to a known method.

**Table 4**

| Preparation of Soft Capsules | |
|---|---|
| Ingredients | Content (Unit: mg) |
| Exosomes derived from freeze-dried milk | 1000 |
| Hyaluronic acid | 75 |
| Collagen peptide | 75 |
| Vitamin C | 75 |
| Vitamin B2 | 4 |
| Vitamin B6 | 3 |
| Tocopherol | 50 |
| Dietary fiber | 100 |
| Wheat germ oil | 3,500 |
| Beeswax | 500 |
| Wax | 500 |

It was found that exosomes derived commercial milk and colostrum according to an example of the present invention not only show an extremely high yield compared to exosomes derived from regular cultured cells, but also show a storage stability that regular cultured cells do not possess, as well as extremely high skin cell regeneration capability. Accordingly, the milk- or colostrum-derived exosomes according to an example of the present invention may be used extremely efficiently as a raw material for functional cosmetics for skin regeneration. Although the present invention has been described with reference to the above-described examples, these examples are merely exemplary, and those skilled in the art shall understand that various modifications and equivalent other embodiments are possible therefrom. Therefore, the true scope of technical protection of the present invention should be determined based on the technical concept of the appended claims.

## INDUSTRIAL APPLICABILITY

A cosmetic composition for skin regeneration according to an example of the present invention not only has a skin regeneration effect significantly higher than that of regular exosomes isolated from cell culture media, but also has a low production cost, a significantly high yield, and a high material stability, and thus, can be extremely economically produced. Accordingly, the milk exosomes according to an example of the present invention may be used for wound healing, immune function enhancement, hair loss prevention and preparation of a pharmaceutical composition for hair growth and hair growth promotion, as well as in the preparation of various functional cosmetics for skin wrinkle reduction or whitening.

## Claims

1. A cosmetic composition for skin regeneration, comprising exosomes derived from milk or goat milk as an active ingredient.

2. The cosmetic composition for skin regeneration of claim 1, wherein the milk or goal milk is raw milk, commercial milk or goat milk, or colostrum.

3. A cosmetic composition for wrinkle reduction, comprising exosomes derived from milk or goat milk as an active ingredient.

4. The cosmetic composition for wrinkle reduction of claim 3, wherein the milk or goat milk is raw milk, commercial milk or goat milk, or colostrum.

5. A cosmetic composition for skin whitening, comprising exosomes derived from milk or goat milk as an active ingredient.

6. The cosmetic composition for skin whitening of claim 5, wherein the milk or goal milk is raw milk, commercial milk or goat milk, or colostrum.

7. A functional shampoo for alleviating hair loss, comprising exosomes derived from milk or goat milk as an active ingredient.

8. The functional shampoo for alleviating hair loss of claim 7, wherein the milk or goat milk is raw milk, commercial milk or goat milk, or colostrum.

9. A functional health food, comprising exosomes derived from milk or goat milk as an active ingredient.

10. The functional health food of claim 9, wherein the milk or goat milk is raw milk, commercial milk or goat milk, or colostrum.

11. The functional health food of claim 9 or 10, which is used for alleviating skin hypersensitivity, alleviating immunity, improving gut health, improving skin health, regulating blood sugar level, antioxidant function or improving liver health.

12. A pharmaceutical composition for treating wound, comprising exosomes derived from milk or goat milk as an active ingredient.

13. The pharmaceutical composition of claim 12, wherein the milk or goat milk is raw milk, commercial milk or goat milk, or colostrum.

14. A method of accelerating the regeneration of wounded skin of a subject, the method comprising applying a therapeutically effective amount of exosomes isolated from milk or goat milk to the wounded skin of the subject.

15. The method of claim 14, wherein the milk or goat milk is raw milk, commercial milk or goat milk, or colostrum.

16. A method of isolating exosomes from milk or goat milk, the method comprising:

a first centrifugation step of removing fat and cells from milk or goat milk by centrifugation;
a filter filtration step of filtering the centrifuged milk or goat milk with a strainer having a mesh size of 20 to 60 $\mu$m, to further remove fat and cells therefrom;
a dilution step of diluting the filtered milk or goat milk by adding an equal volume of distilled water thereto;
an isoelectric precipitation step of adjusting the diluted milk or goat milk to a pH of 4 to 5 by adding an acid thereto, thereby precipitating casein in the milk or goat milk;
a secondary centrifugation step of additionally centrifuging the milk or goat milk in which casein has been precipitated, and collecting supernatant therefrom; and
a filtration step of filtering the collected supernatant by a filter of 0.2 $\mu$m.

17. The method of claim 16,
wherein the first centrifugation step is performed under a temperature condition of 4°C, at 4,000 to 6,000 ×g for 20 to 40 minutes.

18. The method of claim 16,
wherein the strainer is a strainer having a mesh size of 30 to 50 $\mu$m.

19. The method of claim 16,
wherein the acid is hydrochloric acid, nitric acid, acetic acid, or sulfuric acid.

20. The method of claim 16,
wherein the second centrifugation step is performed at room temperature, at 4,000 to 6,000 ×g for 15 to 30 minutes.

FIG. 1A

Process for isolating Milk exosome

Colostrum

Filtration 0.8 µm  Filtration 0.45 µm  Filtration 0.2 µm

Milk Exosomes

FIG. 1B

Milk

↓

5,000 ×g 30 min

Collecting intermediate layer by filtering fat and cells with 40 µm strainer

Dilution w/ equal volume of DW

↓

Adjustment of pH to 4.6 w/ 6N HCl

↓

Collection of supernatant by centrifugation at 5,000 ×g RT for 20 min

↓

Filtration (0.2 µm Only)

FIG. 2A

FIG. 2B

## FIG. 3

## FIG. 4

## FIG. 5

**Experimental conditions**: Freezing at -85°C, Thawing at Room Temperature

## FIG. 6

FIG. 7A

## FIG. 7B

## FIG. 7C

FIG. 8

FIG. 9

FIG. 10

FIG. 11A

w/o UV

Control

H₂O₂

w/ UV

Saline

HaCaT Exo

Comm M-exo

Col M-exo

200μm

**FIG. 11B**

**FIG. 12A**

## FIG. 12B

HDF(Dermal Fibroblast)

FIG.

## FIG. 14

Control    Serum Exo    Comm M-exo    Col M-exo

b)

c)

## FIG. 15A

**FIG. 15B**

**FIG. 15C**

FIG. 16

## FIG. 17

| Control | Serum Exo | Comm M-exo | Col M-exo | Col M-exo-D4 |

## FIG. 18A

DAPI
Collagen I

200 µm

| Saline | HDF Exo | Comm M-exo | Col M-exo |

## FIG. 18B

MMP-2

β-actin

| Saline | HDF Exo | Comm M-exo | Col M-exo |

FIG. 19

FIG. 20

a)

b)

**FIG. 21**

a)

Control  Comm M-exo  Col M-exo

b)

FIG. 22A

FIG. 22B

**DP cell proliferation**

Comm M-exo (µg/ml)

**DP cell proliferation**

DHT 100 µM

Comm M-exo (µg/ml)

## FIG. 22C

DAY1     DAY9     DAY13

PBS 100 µl

Comm M-exo
200 µg/100 µl
(every other day

**INTERNATIONAL SEARCH REPORT**

| International application No. |
|---|
| **PCT/KR2020/017151** |

| A. | CLASSIFICATION OF SUBJECT MATTER |
|---|---|

**A61K 8/98**(2006.01)i; **A61Q 19/00**(2006.01)i; **A61Q 19/08**(2006.01)i; **A61Q 19/02**(2006.01)i; **A61Q 5/02**(2006.01)i; **A61Q 7/00**(2006.01)i; **A23L 33/10**(2016.01)i; **A61K 35/20**(2006.01)i; **A61P 17/02**(2006.01)i

According to International Patent Classification (IPC) or to both national classification and IPC

| B. | FIELDS SEARCHED |
|---|---|

Minimum documentation searched (classification system followed by classification symbols)

A61K 8/98(2006.01); A23L 33/10(2016.01); A23L 33/13(2016.01); A61K 35/20(2006.01); A61K 8/02(2006.01); A61K 8/14(2006.01); A61K 9/00(2006.01); A61P 29/00(2006.01); A61Q 19/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 우유 (milk), 엑소좀 (exosome), 피부 (skin), 화장료 (cosmetics), 주름 (wrinkle), 미백 (whitening), 탈모 (alopecia), 창상 (wound)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | ARDEAZABAL, L. et al. Human breast milk exosomes accelerate mouse wound healing. HUMAN GENE THERAPY. 22-25 October 2019, vol. 30, no. 11, A178, poster P543. See entire document. | 9-13 |
| A | | 1-8,16-20 |
| Y | KR 10-2014-0069569 A (CHANG, Yoo-Min et al.) 10 June 2014 (2014-06-10) | 1-4 |
| X | EP 3192518 A1 (MORINAGA MILK INDUSTRY CO., LTD.) 19 July 2017 (2017-07-19) See abstract; and paragraph [0046]. | 16-20 |
| Y | | 1-4 |
| A | KR 10-2016-0086253 A (INDUSTRY-UNIVERSITY COOPERATION FOUNDATION HANYANG UNIVERSITY ERICA CAMPUS) 19 July 2016 (2016-07-19) See abstract; paragraphs [0014] and [0038]; and claims 1-5. | 1-13,16-20 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 April 2021** | **26 April 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/017151** |

**C. DOCUMENTS CONSIDERED TO BE RELEVANT**

| Category* | Citation of document, with indication, where appropriate, of the relevant passages | Relevant to claim No. |
| --- | --- | --- |
| A | US 2017-0135915 A1 (LIM, S. K. et al.) 18 May 2017 (2017-05-18)<br>See abstract; paragraph [0043]; and claim 1. | 1-13,16-20 |
| PX | KR 10-2020-0124896 A (UNIVERSITY-INDUSTRY COOPERATION GROUP OF KYUNG HEE UNIVERSITY) 04 November 2020 (2020-11-04)<br>See abstract; and claims 1-8. | 5 |

Form PCT/ISA/210 (second sheet) (July 2019)

## INTERNATIONAL SEARCH REPORT

| International application No. |
|---|
| **PCT/KR2020/017151** |

Box No. II      Observations where certain claims were found unsearchable (Continuation of item 2 of first sheet)

This international search report has not been established in respect of certain claims under Article 17(2)(a) for the following reasons:

1. ☑   Claims Nos.: **14-15**
   because they relate to subject matter not required to be searched by this Authority, namely:

   Claims 14-15 pertain to a method for treatment of the human body, and thus pertain to a subject matter on which the International Searching Authority is not required to carry out an international search under the provisions of PCT Article 17(2)(a)(i) and PCT Rule 39.1(iv).

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2020/017151**

| Patent document cited in search report | | | Publication date (day/month/year) | Patent family member(s) | | | Publication date (day/month/year) |
|---|---|---|---|---|---|---|---|
| KR | 10-2014-0069569 | A | 10 June 2014 | None | | | |
| EP | 3192518 | A1 | 19 July 2017 | AU | 2015-313211 | A1 | 02 March 2017 |
| | | | | AU | 2015-313211 | B2 | 18 October 2018 |
| | | | | EP | 3192518 | A4 | 28 March 2018 |
| | | | | EP | 3192518 | B1 | 20 January 2021 |
| | | | | JP | 6349405 | B2 | 27 June 2018 |
| | | | | NZ | 728949 | A | 28 September 2018 |
| | | | | PH | 12017500386 | A1 | 17 July 2017 |
| | | | | WO | 2016-039356 | A1 | 17 March 2016 |
| KR | 10-2016-0086253 | A | 19 July 2016 | KR | 10-1663912 | B1 | 10 October 2016 |
| US | 2017-0135915 | A1 | 18 May 2017 | EP | 2629782 | A1 | 28 August 2013 |
| | | | | EP | 2629782 | B1 | 20 February 2019 |
| | | | | EP | 3563858 | A1 | 06 November 2019 |
| | | | | JP | 2013-540150 | A | 31 October 2013 |
| | | | | JP | 5911874 | B2 | 27 April 2016 |
| | | | | SG | 189438 | A1 | 31 May 2013 |
| | | | | SG | 2014010706 | A | 29 May 2014 |
| | | | | US | 2013-0209528 | A1 | 15 August 2013 |
| | | | | US | 2015-0024011 | A1 | 22 January 2015 |
| | | | | WO | 2012-053976 | A1 | 26 April 2012 |
| KR | 10-2020-0124896 | A | 04 November 2020 | KR | 10-2186872 | B1 | 04 December 2020 |
| | | | | WO | 2020-218846 | A1 | 29 October 2020 |

Form PCT/ISA/210 (patent family annex) (July 2019)

## REFERENCES CITED IN THE DESCRIPTION

### Patent documents cited in the description

- KR 2039302
- KR 1662405
- KR 1894229
- WO 2016039356 A1
- KR 1888258
- KR 1422690
- KR 1663912

### Non-patent literature cited in the description

- **MUNAGALA et al.** *Cancer Lett.,* 2016, vol. 371 (1), 48-61
- **STEED, D. L. et al.** *Clin. Plast. Surg.,* 1998, vol. 25, 397
- Remington's Pharmaceutical Science. Mack Publishing Company
- Remington's Pharmaceutical Science. Mack Publishing Company, 1975
- **YAMAUCHI et al.** *Drug Dev. Ind. Pharm.,* 2019, vol. 45 (3), 359-364